# EUROPÄISCHE PATENTSCHRIFT

(11) **EP 2 683 705 B1**
(45) Veröffentlichungstag und Bekanntmachung des Hinweises auf die Patenterteilung: **22.04.2015**
(21) Anmeldenummer: 12707340.1
(22) Anmeldetag: 07.03.2012
(51) Int. Cl.: C07D 291/08, A61K 31/54, A61P 31/00

(54) **DI- UND TRISUBSTITUIERTE OXATHIAZINDERIVATE, VERFAHREN ZU DEREN HERSTELLUNG, IHRE VERWENDUNG ALS MEDIKAMENT SOWIE SIE ENTHALTENDES ARZNEIMITTEL UND DEREN VERWENDUNG**
DI- AND TRI-SUBSTITUTED OXATHIAZINE DERIVATES, METHOD FOR THE PRODUCTION THEREOF, USE THEREOF AS MEDICINE AND DRUG CONTAINING SAID DERIVATIVES AND USE THEREOF
DÉRIVÉS OXATHIAZINE DI- ET TRI-SUBSTITUÉS, PROCÉDÉ POUR LEUR PRÉPARATION, UTILISATION EN TANT QUE MÉDICAMENT, AGENT PHARMACEUTIQUE CONTENANT CES DÉRIVÉS ET UTILISATION

(30) Priorität: 08.03.2011 EP 11305242
(43) Veröffentlichungstag der Anmeldung: 15.01.2014
(73) Patentinhaber: SANOFI, 75008 Paris (FR)
(72) Erfinder: BOEHME, Thomas, 65926 Frankfurt am Main (DE); ENGEL, Christian, 65926 Frankfurt am Main (DE); GUESSREGEN, Stefan, 65926 Frankfurt am Main (DE); HAACK, Torsten, 65926 Frankfurt am Main (DE); RITTER, Kurt, 65926 Frankfurt am Main (DE); TSCHANK, Georg, 65926 Frankfurt am Main (DE)
(74) Vertreter: Essler, Frank
(86) Internationale Anmeldenummer: PCT/EP2012/053937
(87) Internationale Veröffentlichungsnummer: WO 2012/120054

(56) Entgegenhaltungen:
- US-A- 4 897 405

## Beschreibung

Die Erfindung betrifft substituierte Oxathiazinderivate und deren physiologisch verträgliche Salze.

US 4,897,405 beschreibt 1,2,4,5-Oxathiadiazole 2-oxide-Derivate zur Diabetesbehandlung. Der Erfindung lag die Aufgabe zugrunde, Verbindungen zur Verfügung zu stellen, die eine therapeutisch verwertbare Wirkung entfalten. Insbesondere bestand die Aufgabe darin, neue Verbindungen zu finden, die zur Behandlung von Diabetes, Hyperglykämie, Insulin Resistenz, Adipositasoder Lipidstoffwechselstörungen geeignet sind.

Die Erfindung betrifft daher die Verbindung der Formel I 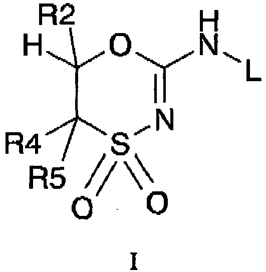 worin bedeuten
- L: R1, -CH(R10)(R11);
- R10, R11: unabhängig voneinander H, F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₁-C₆)-Alkylen-(R6), (C₃-C₈)-Cycloalkylen-(R6), (C₁-C₆)-Alkylen-(C₃-C₈)-Cycloalkylen-(R6), (C₆-C₁₀)-Aryl, (C₁-C₆)-Alkylen-(C₆-C₁₀)-Aryl, -(C₆-C₁₀)-Heteroaryl, (C₁-C₆)-Alkylen-(C₆-C₁₀)-Heteroaryl;
wobei der Arylrest oder Heteroarylrest ein bis 3-fach substituiert sein kann mit F, Cl, Br, I, OH, CF₃, CHF₂, CH₂F, NO₂, CN, OCF₃, OCHF₂, O-(C1-C6)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)- Alkyl)₂, SF₅;
- R6: OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R1: (C₆-C₁₀)-Aryl, (C₃-C₈)-Cycloalkyl, (C₃-C₈)-Carbocyclyl,
wobei der Arylrest, Cycloalkylrest oder Carbocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R2, R4, R5: unabhängig voneinander (C₁-C₆)-Alkyl, CF₃, CHF₂, CH₂F, (C₁-C₆)-Alkylen-OH, (C₁-C₆)-Alkylen-O-(C₁-C₆)-Alkyl, (C₆-C₁₀)-Aryl, (C₁-C₆)-Alkylen-O-(C₆-C₁₀)-Aryl, oder R4 und R5 bilden gemeinsam mit dem Kohlenstoffatom an das sie gebunden sind einen 3-8 gliedrigen, gesättigten carbocyclischen oder heterocyclischen Ring, der bis zu 2 weitere Heteroatome aus der Gruppe N, O oder S enthalten kann;

sowie deren pharmazeutisch verträgliche Salze.

Bevorzugt sind Verbindungen der Formel I, worin bedeuten
- L: R1;
- R1: (C₆-C₁₀)-Ary1, (C₃-C₈)-Cycloalkyl, (C₃-C₈)-Carbocyclyl,
wobei der Arylrest, Cycloalkylrest oder Carbocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R2, R4, R5: unabhängig voneinander (C₁-C₆)-Alkyl, CF₃, CHF₂, CH₂F, (C₁-C₆)-Alkylen-OH, (C₁-C₆)-Alkylen-O-(C₁-C₆)-Alkyl, (C₆-C₁₀)-Aryl, (C₁-C₆)-Alkylen-O-(C₆-C₁₀)-Aryl, oder R4 und R5 bilden gemeinsam mit dem Kohlenstoffatom an das sie gebunden sind einen 3-8 gliedrigen, gesättigten carbocyclischen oder heterocyclischen Ring, der bis zu 2 weitere Heteroatome aus der Gruppe N, O oder S enthalten kann;
sowie deren pharmazeutisch verträgliche Salze.

Weiter bevorzugt sind Verbindungen der Formel I, worin bedeuten
- L: R1;
- R1: (C₆-C₁₀)-Aryl, (C₃-C₈)-Cycloalkyl,
wobei der Arylrest oder Cycloalkylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N(C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R2, R4, R5: unabhängig voneinander (C₁-C₆)-Alkyl, CF₃, CHF₂, CH₂F, (C₁-C₆)-Alkylen-OH, (C₁-C₆)-Alkylen-O-(C₁-C₆)-Alkyl, (C₆-C₁₀)-Aryl, (C₁-C₆)-Alkylen-O-(C₆-C₁₀)-Aryl, oder R4 und R5 bilden gemeinsam mit dem Kohlenstoffatom an das sie gebunden sind einen 3-8 gliedrigen, gesättigten carbocyclischen Ring;
sowie deren pharmazeutisch verträgliche Salze.

Weiter bevorzugt sind Verbindungen der Formel I, worin bedeuten
- L: R1;
- R1: (C₃-C₈)-Cycloalkyl,
wobei der Cycloalkylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, Nl(C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R2: (C₁-C₆)-Alkyl, Phenyl;
- R4, R5: unabhängig voneinander (C₁-C₆)-Alkyl, oder R4 und R5 bilden gemeinsam mit dem Kohlenstoffatom an das sie gebunden sind einen 3-8 gliedrigen, gesättigten carbocyclischen Ring;
sowie deren pharmazeutisch verträgliche Salze.

Können Reste oder Substituenten mehrfach in den Verbindungen der Formeln I auftreten (wie z.B. R6), so können sie alle unabhängig voneinander die angegebene Bedeutung haben und gleich oder verschieden sein.

Die Erfindung bezieht sich auf Verbindungen der Formel I, in Form ihrer Tautomere, Racemate, racemischen Mischungen, Stereoisomerengemische, reinen Stereoisomere, Diastereoisomerengemische, reinen Diastereoisomere. Die Trennung der Gemische erfolgt zum Beispiel auf chromatographischem Weg.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate wie hierin beschrieben.

Unter einem Alkylrest wird eine geradkettige oder verzweigte Kohlenwasserstoffkette verstanden, die eine freie Valenzen aufweist, wie z.B. Methyl, Ethyl, iso-Propyl, tert.-Butyl, Hexyl, Heptyl, Octyl. Die Alkylreste können einfach oder mehrfach wie oben beschrieben substituiert sein.

Unter einem Alkylenrest wird eine geradkettige oder verzweigte Kohlenwasserstoffkette verstanden, die zwei freie Valenzen aufweist, wie z.B. Methylen, Ethylen, iso-Propylen, tert.-Butylen, Hexylen, Heptylen, Octylen. Die Alkylenreste können einfach oder mehrfach wie oben beschrieben substituiert sein.

Unter einem Carbozyklus bzzw. Carbocyclylrest wird ein Ring, welcher gesättigt oder partiell ungesättigt (mit einer oder zwei Doppelbindungen) vorliegt, verstanden, der ausschließlich aus Kohlenstoffatomen aufgebaut ist.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen wie oben beschrieben substituiert sein.,

Unter Heterocyclus bzw. heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterocyclische Rest mit einem weiteren Ring oder Ringsystem anelliert ist. Der Heterocylus bzw. der heterocyclische Rest kann gesättigt, teilweise gesättigt oder aromatisch sein.

Geeignete "Heterocyclen" bzw. "heterocyclische Reste" sind Acridinyl, Azepanyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, 5,6-Dihydro-4H-cyclopentathiazol-2-yl, 4,5-Dihydro-thiazol-2-yl, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, 4,5,6,7-Tetrahydrobenzooxazol-2-yl, 4,5,6,7-Tetrahydro-benzothiazol-2-yl, 4,5,6,7-Tetrahydro-benzoimidazol-2-yl, 4,5,6,7-Tetrahydro-pyrazolo[1,5-a]pyridin-2-yl, Tetrahydrofuranyl, Tetrahydropyranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazinyl, Triazolyl, Tetrazolyl, Thiazolo[4,5-b]pyridinyl, Thieno[2,3-d]thiazol-2-yl, Tropanyl und Xanthenyl.

Die Heterocyclen bzw. heterocyclischen Reste können ein oder mehrfach mit geeigneten Gruppen wie oben beschrieben substituiert sein.,

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacryl säuremethyl ester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im Allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:
Alle Antidiabetika, die in der Roten Liste 2009, Kapitel 12 genannt sind; alle Abmagerungsmittel/Appetitzügler, die in der Roten Liste 2009, Kapitel 1 genannt sind; alle Diuretika, die in der Roten Liste 2009, Kapitel 36 genannt sind; alle Lipidsenker, die in der Roten Liste 2009, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Erfolgt die Gabe der Wirkstoffe durch getrennte Verabreichung der Wirkstoffe, so kann diese gleichzeitig oder nacheinander erfolgen. Die meisten der nachfolgend aufgeführten Wirkstoffe sind in USP Dictionary of USAN and International Drug Names, US Pharmacopeia, Rockville 2006, offenbart.

Antidiabetika umfassen Insulin und Insulinderivate, wie z.B. Lantus^{®} (siehe www.lantus.com) oder HMR 1964 oder Levemir® (insulin detemir), Humalog^{(R)} (Insulin Lispro), Humulin^{(R)}, VIAject™ oder solche, wie sie in WO2005005477 (Novo Nordisk) beschrieben sind, schnell wirkende Insuline (siehe US 6,221,633), inhalierbare Insuline, wie z. B. Exubera ^{®}, Nasulin™, oder orale Insuline, wie z. B. IN-105 (Nobex) oder Oral-lyn ™ (Generex Biotechnology) oder Technosphere^{(R)} Insulin (MannKind) oder Cobalamin™ orales Insulin oder Insuline, wie sie in WO2007128815, WO2007128817 beschrieben sind oder Insuline, die transdermal verabreicht werden können;

GLP-1-Derivate und GLP-1 Agonisten wie z.B. Exenatide, Liraglutide oder diejenigen die in WO 98/08871, WO2005027978, WO2006037811, WO2006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), AVE-0010, BIM-51077 (R-1583, ITM-077), PC-DAC:Exendin-4 (ein Exendin-4 Analogon, welches kovalent an rekombinantes menschliches Albumin gebunden ist), CVX-73, CVX-98 und CVx-96 (GLP-1 Analoga, welche kovalent an einen monoklonalen Antikörper gebunden sind, der spezifische Bindungsstellen für das GLP-1 Peptid aufweist), CNTO-736 (ein GLP-1 Analogon, welches an eine Domäne gebunden ist, welche den Fc-Teil eines Antikörpers beinhaltet), PGC-GLP-1 (GLP-1 gebunden an einen Nanocarrier), Agonisten wie sie z.B. bei D. Chen et al., Proc. Natl. Acad. Sci. USA 104 (2007) 943 beschrieben sind, solche wie sie in WO2006124529, WO2007124461 beschrieben sind, Peptide wie z.B. Obinepitide (TM-30338), Amylinrezeptor Agonisten, wie sie z.B. in WO2007104789 beschrieben sind, Analoga des humanen GLP-1, wie sie in WO2007120899 beschrieben sind, sowie oral wirksame hypoglykämische Wirkstoffe.

Antidiabetika umfassen auch Agonisten des Glukose-abhängigen insulinotropen Polypeptids (GIP) Rezeptors wie sie z.B. in WO2006121860 beschrieben sind.

Antidiabetika umfassen auch Analoga und Derivate des Fibroblastenwachstumsfaktors 21 (FGF-21, fibroblast growth factor 21).

Die oral wirksamen hypoglykämischen Wirkstoffe umfassen vorzugsweise Sulfonylharnstoffe,
Biguanidine,
Meglitinide,
Oxadiazolidindione,
Thiazolidindione,
PPAR- und RXR-Modulatoren,
Glukosidase-Inhibitoren,
Hemmstoffe der Glykogenphosphorylase,
Glukagonrezeptor-Antagonisten,
Glukokinaseaktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT),
GLP-1-Agonisten,
Kaliumkanalöffner, wie z.B. Pinacidil, Cromakalim, Diazoxid oder solche wie sie bei R. D. Carr et al., Diabetes 52, 2003, 2513.2518, bei J. B. Hansen et al, Current Medicinal Chemistry 11, 2004, 1595-1615, bei T. M. Tagmose et al., J. Med. Chem. 47, 2004, 3202-3211 oder bei M. J. Coghlan et al., J. Med. Chem. 44, 2001, 1627-1653 beschrieben sind, oder diejenigen, die in WO 97/26265 und WO 99/03861 von Novo Nordisk A/S offenbart wurden,
Wirkstoffe, die auf den ATP-abhängigen Kaliumkanal der Betazellen wirken,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption,
Modulatoren der natrium-abhängigen Glukosetransporter 1 oder 2 (SGLT1, SGLT2),
Hemmstoffe der 11-beta-Hydroxysteroid-Dehydrogenase-1 (11ß-HSD1),
Inhibitoren der Protein-Tyrosin-Phosphatase-1B (PTP-1B),
Nikotinsäurerezeptoragonisten,
Inhibitoren der hormon-sensitiven bzw. endothelialen Lipasen,
Hemmstoffen der Acetyl-CoA Carboxylase (ACC1 und/oder ACC2) oder Inhibitoren der GSK-3 beta.
Weiterhin sind umfasst den Fettstoffwechsel verändernde Verbindungen wie antihyperlipidämische Wirkstoffe und antilipidämische Wirkstoffe,
HMGCoA-Reduktase-Inhibitoren,
Farnesoid X Rezeptor (FXR) Antagonisten,
Fibrate,
Cholesterinresreptionsinhibitoren,
CETP-Inhibitoren,
Gallensäureresorptionsinhibitoren,
MTP-Inhibitoren,
Agonisten des Estrogenrezeptors gamma (ERR Agonisten),
Sigma-1 Rezeptorantagonisten,
Antagonisten des Somatostatin 5 Rezeptors (SST5 Rezeptor);
Verbindungen, die die Nahrungsmitteleinnahme verringern und
Verbindungen, die die Thermogenese erhöhen.

Bei einer Ausführungsform der Erfindung wird die Verbindung der Formel I in Kombination mit Insulin verabreicht.

Bei einer Ausführungsform wird die Verbindung der Formel I in Kombination mit einem Wirkstoff, der auf den ATP-abhängigen Kaliumkanal der Betazellen wirkt, z.B. Sulfonylharnstoffe, wie z.B. Tolbutamid, Glibenclamid, Glipizid, Gliclazide oder Glimepirid, verabreicht.

Bei einer Ausführungsform wird die Verbindung der Formel I in Kombination mit einer Tablette verabreicht, die sowohl Glimeprid enthält, welches schnell freigesetzt wird wie auch Metformin enthält, welches über einen längeren Zeitraum freigesetzt wird (wie z.B. in US2007264331 beschrieben).

Bei einer Ausführungsform wird die Verbindung der Formel I in Kombination mit einem Biguanid, wie z.B. Metformin, verabreicht.

Bei wieder einer Ausführungsform wird die Verbindung der Formel I in Kombination mit einem Meglitinid, wie z.B. Repaglinide, Nateglinid oder Mitiglinide verabreicht.

Bei einer weiteren Ausführungsform wird die Verbindung der Formel I mit einer Kombination von Mitiglinide mit einem Glitazon, z.B. Pioglitazon Hydrochlorid, verabreicht.

Bei einer weiteren Ausführungsform wird die Verbindung der Formel I mit einer Kombination von Mitiglinide mit einem alpha-Glukosidaseinhibitor verabreicht.

Bei einer weiteren Ausführungsform wird die Verbindung der Formel I in Kombination mit antidiabetischen Verbindungen, wie sie in WO2007095462, WO2007101060, WO2007105650 beschrieben sind, verabreicht.

Bei einer weiteren Ausführungsform wird die Verbindung der Formel I in Kombination mit antihypoglykämischen Verbindungen, wie sie in WO2007137008 beschrieben sind, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483, CS-011 (Rivoglitazon), DRL-17564, DRF-2593 (Balaglitazon) oder solchen, wie sie in WO2007060992, WO2007100027, WO2007103252, WO2007122970 beschrieben sind, verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.

Bei einer Ausführungsform der Erfindung wird die Verbindung der Formel I in Kombination mit Tandemact™, einer festen Kombination von Pioglitazon mit Glimeprid, verabreicht.

Bei einer weiteren Ausführungsform der Erfindung wird die Verbindung der Formel I in Kombination mit einer festen Kombination von Pioglitazon Hydrochlorid mit einem Angiotensin II Agonisten, wie z.B. TAK-536, verabreicht.

Bei einer Ausführungsform der Erfindung wird die Verbindung der Formel I in Kombination mit einem PPAR alpha Agonisten bzw. gemischten PPAR alpha/PPAR delta Agonisten, wie z.B. GW9578, GW-590735, K-111, LY-674, KRP-101, DRF-10945, LY-518674, CP-900691, BMS-687453, BMS-711939 oder solchen wie sie in WO2001040207, WO2002096894, WO2005097076, WO2007056771, WO2007087448, WO2007089667, WO2007089557, WO2007102515, WO2007103252, JP2007246474, WO2007118963, WO2007118964, WO2007126043 beschrieben sind, verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, CKD-501 (Lobeglitazon Sulfat), MBX-213 oder wie in WO 00/64888, WO 00/64876, WO03/020269, WO2007099553, US2007276041, WO2007085135, WO2007085136 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 oder wie sie in WO2006059744, WO2006084176, WO2006029699, WO2007039172-WO2007039178, WO2007071766, WO2007101864, US2007244094, WO2007119887 beschrieben sind, verabreicht.

Bei einer Ausführungsform der Erfindung wird die Verbindung der Formel I in Kombination mit einem pan-SPPARM (selective PPAR modulator alpha, gamma, delta), wie z.B. GFT-505, verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht.

Bei einer Ausführungsform wird die Verbindung der Formel I in Kombination mit einem α-Glukosidase-Inhibitor, wie z.B. Miglitol oder Acarbose oder solchen, wie sie z.B. in WO2007114532, WO2007140230 beschrieben sind, verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932 beschrieben, verabreicht.

Bei einer Ausführungsform wird die Verbindung der Formel I in Kombination mit GlukagonRezeptor-Antagonisten, wie z.B. A-770077 oder NNC-25-2504 oder wie in WO2004100875, WO2005065680, WO2006086488, WO2007106181, WO2007111864, WO2007120270, WO2007120284, WO2007123581, WO2007136577 beschrieben, verabreicht.

Bei einer weiteren Ausführungsform wird die Verbindung der Formel I in Kombination mit einer Antisense-Verbindung, z.B. ISIS-325568, verabreicht, welche die Produktion des Glukagonrezeptors inhibiert.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031, WO2004072066, WO2005080360, WO2005044801, WO2006016194, WO2006058923, WO2006112549, WO2006125972, WO2007017549, WO2007017649, WO2007007910, WO2007007040-42, WO2007006760-61, WO2007006814, WO2007007886, WO2007028135, WO2007031739, WO2007041365, WO2007041366, WO2007037534, WO2007043638, WO2007053345, WO2007051846, WO2007051845, WO2007053765, WO2007051847, WO2007061923, WO2007075847, WO2007089512, WO2007104034, WO2007117381, WO2007122482, WO2007125103, WO2007125105, US2007281942 beschrieben sind, verabreicht.

Bei einer Ausführungsform wird die Verbindung der Formel I in Kombination mit einem Inhibitor der Glukoneogenese, wie z. B. FR-225654, verabreicht.

Bei einer Ausführungsform wird die Verbindung der Formel I in Kombination mit Inhibitoren der Fructose-1,6-bisphosphatase (FBPase) wie z.B. CS-917 (MB-06322) oder MB-07803 oder solchen wie sie in WO2006023515, WO2006104030, WO2007014619, WO2007137962 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Sitagliptin Phosphat, Saxagliptin ((BMS-477118), GSK-823093, PSN-9301, SYR-322, SYR-619, TA-6666, TS-021, GRC-8200, GW-825964X, KRP-104, DP-893, ABT-341, ABT-279 oder ein anderes Salz davon, S-40010, S-40755, PF-00734200, BI-1356, PHX-1149, Alogliptin oder solchen Verbindungen wie sie in WO2003074500, WO2003106456, WO2004037169, WO200450658, WO2005037828, WO2005058901, WO2005012312, WO2005/012308, WO2006039325, WO2006058064, WO2006015691, WO2006015701, WO2006015699, WO2006015700, WO2006018117, WO2006099943, WO2006099941, JP2006160733, WO2006071752, WO2006065826, WO2006078676, WO2006073167, WO2006068163, WO2006085685, WO2006090915, WO2006104356, WO2006127530, WO2006111261, WO2007015767 (LY-2463665), WO2007024993, WO2007029086, WO2007063928, WO2007070434, WO2007071738, WO2007077508, WO2007087231, WO2007097931, WO2007099385, WO2007100374, WO2007112347, WO2007112669, WO2007113226, WO2007113634, WO2007115821, WO2007116092, US2007259900, EP1852108, US2007270492, WO2007126745, WO2007136603 beschrieben sind, verabreicht.

Bei einer Ausführungsform wird die Verbindung der Formel I in Kombination mit Janumet™, einer festen Kombination von Sitagliptin Phosphat mit Metformin Hydrochlorid, verabreicht.

Bei einer Ausführungsform wird die Verbindung der Formel I in Kombination mit Eucreas^{(R)}, einer festen Kombination von Vildagliptin mit Metformin Hydrochlorid, verabreicht.

Bei einer Ausführungsform wird die Verbindung der Formel I in Kombination mit einer Kombination eines DPP-IV-Inhibitors mit omega-3-Fettsäuren oder omega-3-Fettsäureestern, wie z.B. in WO2007128801 beschrieben, verabreicht.

Bei einer Ausführungsform wird die Verbindung der Formel I in Kombination mit einer die Insulinsekretion verstärkende Substanz, wie z. B. KCP-265 (WO2003097064), oder solchen wie sie in WO2007026761 beschrieben sind, verabreicht.

Bei einer Ausführungsform wird die Verbindung der Formel I in Kombination mit Agonisten des glucose-abhängigen insulinotropischen Rezeptors (GDIR) wie z. B. APD-668 verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268, SAR 7226, SGL-5083, SGL-5085, SGL-5094, ISIS-388626, Sergliflozin oder Dapagliflozin oder wie sie z. B. in WO2004007517, WO200452903, WO200452902, PCT/EP2005/005959, WO2005085237, JP2004359630, WO2005121161, WO2006018150, WO2006035796, WO2006062224, WO2006058597, WO2006073197, WO2006080577, WO2006087997, WO2006108842, WO2007000445, WO2007014895, WO2007080170, WO2007093610, WO2007126117, WO2007128480, WO2007129668, US2007275907, WO2007136116 oder von A. L. Handlon in Expert Opin. Ther. Patents (2005) 15(11), 1531-1540 beschrieben sind, verabreicht.

Bei einer Ausführungsform wird die Verbindung der Formel I in Kombination mit Hemmstoffen der 11-beta-Hydroxysteroid-Dehydrogenase-1 (11ß-HSD1), wie z. B. BVT-2733, JNJ-25918646, INCB-13739, DIO-92 ((-)-Ketoconazol) oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005063247, WO!005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109, WO2006074244, WO2006078006, WO2006106423, WO2006132436, WO2006134481, WO2006134467, WO2006135795, WO2006136502, WO2006138508, WO2006138695, WO2006133926, WO2007003521, WO2007007688, US2007066584, WO2007029021, WO2007047625, WO2007051811, WO2007051810, WO2007057768, WO2007058346, WO2007061661, WO2007068330, WO2007070506, WO2007087150, WO2007092435, WO2007089683, WO2007101270, WO2007105753, WO2007107470, WO2007107550, WO2007111921, US2007207985, US2007208001, WO2007115935, WO2007118185, WO2007122411, WO2007124329, WO2007124337, WO2007124254, WO2007127688, WO2007127693, WO2007127704, WO2007127726, WO2007127763, WO2007127765, WO2007127901, US2007270424, JP2007291075, WO2007130898, WO2007135427 beschrieben sind, verabreicht.

Bei einer Ausführungsform wird die Verbindung der Formel I in Kombination mit Inhibitoren der Protein-Tyrosin-Phosphatase-1B (PTP-1B), wie sie z. B. in WO200119830-31, WO200117516, WO2004506446, WO2005012295, WO2005116003, WO2005116003, WO2006007959, DE 10 2004060542.4, WO2007009911, WO2007028145, WO2007067612-615, WO2007081755, WO2007115058 beschrieben sind, verabreicht.

Bei einer Ausführungsform der Erfindung wird die Verbindung der Formel I in Kombination mit einem Agonisten des GPR109A (HM74A Rezeptor Agonisten; NAR-Agonisten (Nikotinsäurerezeptoragonisten)), wie z.B. Nicotinsäure oder "extended release niacin" in Verbindung mit MK-0524A (Laropiprant) oder MK-0524 oder solchen Verbindungen, wie sie in WO2006045565, WO2006045564, WO2006069242, WO2006085108, WO2006085112, WO2006085113, WO2006124490, WO2006113150, WO2007017261, WO2007017262, WO2007017265, WO2007015744, WO2007027532, WO2007092364, WO2007120575, WO2007134986 beschrieben sind, verabreicht.

Bei einer anderen Ausführungsform der Erfindung wird die Verbindung der Formel I in Kombination mit einer festen Kombination von Niacin mit Simvastatin verabreicht.

Bei einer anderen Ausführungsform der Erfindung wird die Verbindung der Formel I in Kombination mit Nicotinsäure oder "extended release niacin" in Verbindung mit MK-0524A (Laropiprant) verabreicht.

Bei einer weiteren Ausführungsform der Erfindung wird die Verbindung der Formel I in Kombination mit Nicotinsäure oder "extended release niacin" in Verbindung mit MK-0524A (Laropiprant) und mit Simvastatin verabreicht.

Bei einer anderen Ausführungsform der Erfindung wird die Verbindung der Formel I in Kombination mit einem Agonisten des GPR116, wie sie z.B. in WO2006067531, WO2006067532 beschrieben sind, verabreicht.

Bei einer Ausführungsform wird die Verbindung der Formel I in Kombination mit Modulatoren des GPR40, wie sie z.B. in WO2007013689, WO2007033002, WO2007106469, US2007265332, WO2007123225, WO2007131619, WO2007131620, WO2007131621, US2007265332, WO2007131622, WO2007136572 beschrieben sind, verabreicht.

Bei einer Ausführungsform wird die Verbindung der Formel I in Kombination mit Modulatoren des GPR119b wie sie z. B. in WO2004041274 beschrieben sind, verabreicht.

Bei einer Ausführungsform wird die Verbindung der Formel I in Kombination mit Modulatoren des GPR119 (G-Protein-gekoppelter Glukose-abhängiger insulinotroper Rezeptor), wie z.B. PSN-119-1 oder solchen wie sie z. B. in WO2005061489 (PSN-632408), WO2004065380, WO2006018662, WO2007003960-62 und WO2007003964, WO2007116229, WO2007116230 beschrieben sind, verabreicht.

Bei einer weiteren Ausführungsform wird die Verbindung der Formel I in Kombination mit Modulatoren des GPR120, wie sie z.B. in EP1688138 beschrieben sind, verabreicht.

Bei einer Ausführungsform wird die Verbindung der Formel I in Kombination mit Inhibitoren der hormon-sensitiven Lipase (HSL) und/oder Phospholipasen, wie z. B. in WO2005073199, WO2006074957, WO2006087309, WO2006111321, WO2007042178, WO2007119837 beschrieben, verabreicht.

Bei einer Ausführungsform wird die Verbindung der Formel I in Kombination mit Inhibitoren der endothelialen Lipase, wie z. B. in WO2007110216 beschrieben, verabreicht.

Bei einer Ausführungsform wird die Verbindung der Formel I in Kombination mit einem Phospholipase A2 Inhibitor wie z.B. Darapladib oder A-002 verabreicht.

Bei einer Ausführungsform wird die Verbindung der Formel I in Kombination mit Myricitrin, einem Lipase-Inhibitor (WO2007119827), verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, WO2005111018, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117, WO2007073117, WO2007083978, WO2007120102, WO2007122634, WO2007125109, WO2007125110 beschrieben.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Serum/Glucocorticoid regulierten Kinase (SGK), wie z. B. in WO2006072354, WO2007093264 beschrieben, verabreicht.

Bei einer Ausführungsform wird die Verbindung der Formel I in Kombination mit einem Inhibitor der Protein Kinase C beta (PKC beta), wie z. B. Ruboxistaurin, verabreicht.

Bei einer weiteren Ausführungsform wird die Verbindung der Formel I in Kombination mit einem Aktivator der AMP-aktivierten Proteinkinase (AMPK), wie sie z. B. in WO2007062568 beschrieben sind, verabreicht.

Bei einer Ausführungsform wird die Verbindung der Formel I in Kombination mit einem Inhibitor der Ceramidkinase, wie sie z. B. in WO2007112914 beschrieben sind, verabreicht.

Bei einer weiteren Ausführungsform wird die Verbindung der Formel I in Kombination mit einem Inhibitor der MAPK-interagierenden Kinase 2 (MNK2), wie sie z.B. in WO2007104053, WO2007115822 beschrieben sind, verabreicht.

Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022057, WO2004022553, WO2005097129, WO2005113544, US2007244140 beschrieben sind, verabreicht.

Bei einer Ausführungsform der Erfindung wird die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin, L-659699 oder solchen, wie sie in US2007249583 beschrieben sind, verabreicht.

Bei einer weiteren Ausführungsform der Erfindung wird die Verbindung der Formel I in Kombination mit einem Farnesoid X Rezeptor (FXR) Antagonisten, wie z.B. in WO2007052843, WO2007070796, WO2007092751, JP2007230909, WO2007095174, WO2007140174, WO200714018 beschrieben, verabreicht.

Bei einer anderen Ausführungsform der Erfindung wird die Verbindung der Formel I in Kombination mit einem Liganden des Leber X Rezeptors (liver X receptor; LXR), wie z.B. in WO2007092965 beschrieben, verabreicht.

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit Fibraten, wie z.B. dem Cholinsalz von Fenofibrat (SLV-348), verabreicht.

Bei einer Ausführungsform der Erfindung wird die Verbindung der Formel I in Kombination mit Fibraten, wie z.B. dem Cholinsalz von Fenofibrat und einem HMGCoA Reduktase Inhibitor, wie z.B. Rosuvastatin, verabreicht.

Bei einer weiteren Ausführungsform der Erfindung wird die Verbindung der Formel I in Kombination mit Bezafibrat und Diflunisal verabreicht.

Bei einer weiteren Ausführungsform der Erfindung wird die Verbindung der Formel I in Kombination mit einer festen Kombination von Fenofibrat oder einem Salz davon mit Simvastatin, Rosuvastatin, Fluvastatin, Lovastatin, Cerivastatin, Pravastatin oder Atorvastatin verabreicht.

Bei einer weiteren Ausführungsform der Erfindung wird die Verbindung der Formel I in Kombination mit Synordia (R), einer festen Kombination von Fenofibrat mit Metformin, verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692, WO2005005453), MD-0727 (Microbia Inc., WO2005021497, WO2005021495) oder mit Verbindungen, wie in WO2002066464, WO2005000353 (Kotobuki Pharmaceutical Co. Ltd.) oder WO2005044256 oder WO2005062824 (Merck & Co.) oder WO2005061451 und WO2005061452 (AstraZeneca AB) und WO2006017257 (Phenomix) oder WO2005033100 (Lipideon Biotechnology AG) oder wie in WO2002050060, WO2002050068, WO2004000803, WO2004000804, WO2004000805, WO2004087655, WO2004097655, WO2005047248, WO2006086562, WO2006102674, WO2006116499, WO2006121861, WO2006122186, WO2006122216, WO2006127893, WO2006137794, WO2006137796, WO2006137782, WO2006137793, WO2006137797, WO2006137795, WO2006137792, WO2006138163, WO2007059871, US2007232688, WO2007126358 beschrieben, verabreicht.

Bei einer Ausführungsform der Erfindung wird die Verbindung der Formel I in Kombination mit Vytorin™, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Atorvastatin, verabreicht.

Bei einer Ausführungsform der Erfindung wird die Verbindung der Formel I in Kombination mit einer festen Kombination von Ezetimibe mit Fenofibrat verabreicht.

Bei einer Ausführungsform der Erfindung ist der weitere Wirkstoff ein Diphenylazetidinonderivat, wie z.B. in US 6,992,067 oder US 7,205,290 beschrieben.

Bei einer weiteren Ausführungsform der Erfindung ist der weitere Wirkstoff ein Diphenylazetidinonderivat, wie z.B. in US 6,992,067 oder US 7,205,290 beschrieben, kombiniert mit einem Statin, wie z.B. Simvastatin, Fluvastatin, Pravastatin, Lovastatin, Cerivastatin, Atorvastatin oder Rosuvastatin.

Bei einer Ausführungsform der Erfindung wird die Verbindung der Formel I in Kombination mit einer festen Kombination von Lapaquistat, einem Squalensynthase-Inhibitor, mit Atorvastatin verabreicht.

Bei einer Ausführungsform der Erfindung wird die Verbindung der Formel I in Kombination mit einem CETP-Inhibitor, wie z.B. Torcetrapib, Anacetrapib oder JTT-705 oder solchen wie sie in WO2006002342, WO2006010422, WO2006012093, WO2006073973, WO2006072362, WO2007088996, WO2007088999, US2007185058, US2007185113, US2007185154, US2007185182, WO2006097169, WO2007041494, WO2007090752, WO2007107243, WO2007120621, US2007265252, US2007265304, WO2007128568, WO2007132906 beschrieben sind, verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9, WO2007009655-56 beschrieben, verabreicht.

Bei einer Ausführungsform wird die Verbindung der Formel I in Kombination mit Agonisten des GPBAR1 (G-protein-coupled-bile-acid-receptor-1, TGR5), wie sie z.B. in WO2007110237, WO2007127505 beschrieben sind, verabreicht.

Bei einer Ausführungsform der Erfindung wird die Verbindung der Formel I in Kombination mit einem polymeren Gallensäureadsorber, wie z.B. Cholestyramin, Colesevelam Hydrochlorid, verabreicht.

Bei einer Ausführungsform der Erfindung wird die Verbindung der Formel I in Kombination mit einem Phytosterole enthaltenden Kaugummi (Reductol™) verabreicht.

Bei einer Ausführungsform der Erfindung wird die Verbindung der Formel I in Kombination mit einem Inhibitor des mikrosomalen Triglycerid-Transfer-Proteins (MTP-Inhibitor), wie z.B. Implitapide, BMS-201038, R-103757, AS-1552133, SLx-4090, AEGR-733 oder solchen wie in WO2005085226, WO2005121091, WO2006010423, WO2006113910 beschrieben, verabreicht.

Bei einer anderen Ausführungsform der Erfindung wird die Verbindung der Formel I in Kombination mit einem Antagonisten des Somatostatin 5 Rezeptors (SST5 Rezeptor), wie z.B. solchen wie sie in WO2006094682 beschrieben sind, verabreicht.

Bei einer Ausführungsform der Erfindung wird die Verbindung der Formel I in Kombination mit einem ACAT-Inhibitor, wie z.B. Avasimibe, SMP-797 oder KY-382, verabreicht.

Bei einer weiteren Ausführungsform der Erfindung wird die Verbindung der Formel I in Kombination mit einem Inhibitor der Leber-Carnitin Palmitoyltransferase-1 (L-CPT1), wie sie z.B. in WO2007063012, WO2007096251 (ST-3473) beschrieben sind, verabreicht.

Bei einer Ausführungsform der Erfindung wird die Verbindung der Formel I in Kombination mit einem Squalen Synthetase Inhibitor, wie z.B. BMS-188494, TAK-475 (Lapaquistat Acetat) oder wie in WO2005077907, JP2007022943 beschrieben, verabreicht.

Bei einer Ausführungsform der Erfindung wird die Verbindung der Formel I in Kombination mit ISIS-301012 (Mipomersen), einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in WO2005097738 beschrieben, verabreicht.

Bei einer Ausführungsform der Erfindung wird die Verbindung der Formel I in Kombination mit einem ABCA1 Expressionsverstäker, wie sie z.B. in WO2006072393 beschrieben, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit einem Adenosin A2B Rezeptor Agonisten (Adenosin A2B R) wie z.B. ATL-801 verabreicht.

Bei einer anderen Ausführungsform der Erfindung wird die Verbindung der Formel I in Kombination mit einem Modulator der Adenosin A2A und/oder Adenosin A3 Rezeptoren, wie z.B. in WO2007111954, WO2007121918, WO2007121921, WO2007121923 beschrieben, verabreicht.

Bei einer Ausführungsform der Erfindung wird die Verbindung der Formel I in Kombination mit einem Adenosin A2B Rezeptor Antagonisten (Adenosin A2B R), wie sie in US2007270433 beschrieben sind, verabreicht.

Bei einer Ausführungsform wird die Verbindung der Formel I in Kombination mit Hemmstoffen der Acetyl-CoA Carboxylase (ACC1 und/oder ACC2) wie z. B. solchen wie in WO199946262, WO200372197, WO2003072197, WO2005044814, WO2005108370, JP2006131559, WO2007011809, WO2007011811, WO2007013691, WO2007095601-603, WO2007119833 beschrieben, verabreicht.

Bei einer anderen Ausführungsform wird die Verbindung der Formel I in Kombination mit Modulatoren der mikrosomalen Acyl-CoA:Glycerol-3-Phosphat-Acyltransferase 3 (GPAT3, beschrieben in WO2007100789) oder mit Modulatoren der mikrosomalen Acyl-CoA:Glycerol-3-Phosphat-Acyltransferase 4 (GPAT4, beschrieben in WO2007100833) verabreicht.

Bei einer weiteren Ausführungsform wird die Verbindung der Formel I in Kombination mit Modulatoren der Xanthin-Oxidoreductase (XOR) verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
NPY-5 Rezeptorantagonisten wie L-152804 oder die Verbindung "NPY-5-BY" der Firma Banyu oder wie sie z. B. in WO2006001318, WO2007103295, WO2007125952 beschrieben sind;
NPY-4-Rezeptorantagonisten wie sie z. B. in WO2007038942 beschrieben sind;
NPY-2-Rezeptorantagonisten wie sie z. B. in WO2007038943 beschrieben sind;
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424, WO2006095166 beschrieben sind;
Derivaten des Peptids Obestatin wie sie WO2006096847 beschrieben sind;
CB1R (Cannabinoid Rezeptor 1) Antagonisten (wie z.B. Rimonabant, Surinabant (SR147778), SLV-319, AVE-1625, Taranabant (MK-0364) oder Salze davon, V-24343 oder solche Verbindungen wie sie in z. B. EP 0656354, WO 00/15609, WO2001/64632-64634, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006047516, WO2006060461, WO2006067428, WO2006067443, WO2006087480, WO2006087476, WO2006100208, WO2006106054, WO2006111849, WO2006113704, WO2007009705, WO2007017124, WO2007017126, WO2007018459, WO2007018460, WO2007016460, WO2007020502, WO2007026215, WO2007028849, WO2007031720, WO2007031721, WO2007036945, WO2007038045, WO2007039740, US20070015810, WO2007046548, WO2007047737, WO2007057687, WO2007062193, WO2007064272, WO2007079681, WO2007084319, WO2007084450, WO2007086080, EP1816125, US2007213302, WO2007095513, WO2007096764, US2007254863, WO2007119001, WO2007120454, WO2007121687, WO2007123949, US2007259934, WO2007131219, WO2007133820, WO2007136607, WO2007136571, US7297710, WO2007138050, WO2007140385, WO2007140439 beschrieben sind);
Cannabinoid Rezeptor 1 / Cannabinoid Rezeptor 2 (CB1/CB2) modulierende Verbindungen wie z.B. delta-9-Tetrahydrocannabivarin oder solchen wie sie z.B. in WO2007001939, WO2007044215, WO2007047737, WO2007095513, WO2007096764, WO2007112399, WO2007112402 beschrieben sind;
Modulatoren der FAAH (fatty acid amide hydrolase) wie sie z.B. in WO2007140005 beschrieben sind;
Vanilloid-1-Rezeptor Modulatoren (Modulatoren des TRPV1), wie sie z.B. in WO2007091948, WO2007129188, WO2007133637 beschrieben sind;
Aktivatoren des Capsaicinrezeptors, wie sie z.B. in JP2007210969 beschrieben sind;
Agonisten des Prostaglandinrezeptors, wie z.B. Bimatoprost oder solchen Verbindungen wie sie in WO2007111806 beschrieben sind;
MC4-Rezeptor Agonisten (Melanocortin-4 Rezeptor Agonisten, MC4R Agonisten wie z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chlor-phenyl)-2-oxo-ethyl]-amid; (WO 01/91752)) oder LB53280, LB53279, LB53278 oder THIQ, MB243, RY764, CHIR-785, PT-141, MK-0493 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, WO2005118573, EP1538159, WO2004072076, WO2004072077, WO2006021655-57, WO2007009894, WO2007015162, WO2007041061, WO2007041052, JP2007131570, EP-1842846, WO2007096186, WO2007096763 beschrieben sind;
Orexin-Rezeptor 1 Antagonisten (OX1R Antagonisten), Orexin-Rezeptor 2 Antagonisten (OX2R Antagonisten) oder gemischte OX1R/OX2R Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224, WO2007085718, WO2007088276, WO2007116374, WO2007122591, WO2007126934, WO2007126935 beschrieben sind);
Histamin H3 Rezeptor Antagonisten/inverse Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893, US2005171181 (z.B. PF-00389027), WO2006107661, WO2007003804, WO2007016496, WO2007020213, WO2007049798, WO2007055418, WO2007057329, WO2007065820, WO2007068620, WO2007068641, WO2007075629, WO2007080140, WO2007082840, WO2007088450, WO2007088462, WO2007094962, WO2007099423, WO2007100990, WO2007105053, WO2007106349, WO2007110364, WO2007115938, WO2007131907, WO2007133561, US2007270440, WO2007135111 beschrieben sind);
Histamin H1 / Histamin H3 Modulatoren, wie z. B. Betahistin bzw. seinem Dihydrochlorid;
CRF-Antagonisten (z.B. [2-Methyl-9-(2,4,6-trimethyl-phenyl)-9H-1,3,9-triaza-fluoren-4-yl]-dipropyl-amin (WO 00/66585) oder solche CRF1-Antagonisten, wie sie in WO2007105113, WO2007133756 beschrieben sind);
CRF BP-Antagonisten (z.B. Urocortin);
Urocortin-Agoni sten;
Agonisten des beta-3 Adrenoceptors wie z.B. 1-(4-Chlor-3-methansulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553, WO2002038543, WO2002038544, WO2007048840-843 beschrieben sind;
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2005085200, WO2005019240, WO2004011438, WO2004012648, WO2003015769, WO2004072025, WO2005070898, WO2005070925, WO2004039780, WO2004092181, WO2003033476, WO2002006245, WO2002089729, WO2002002744, WO2003004027, FR2868780, WO2006010446, WO2006038680, WO2006044293, WO2006044174, JP2006176443, WO2006018280, WO2006018279, WO2006118320, WO2006130075, WO2007018248, WO2007012661, WO2007029847, WO2007024004, WO2007039462, WO2007042660, WO2007042668, WO2007042669, US2007093508, US2007093509, WO2007048802, JP2007091649, WO2007092416; WO2007093363-366, WO2007114902, WO2007114916 beschrieben sind);
CCK-A (CCK-1) Agonisten (wie z.B. {2-[4-(4-Chlor-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180) oder solchen, wie sie in WO2005116034, WO2007120655, WO2007120688, WO2007120718 beschrieben sind;
Serotonin-Wiederaufnahme-Inhibitoren (z.B. Dexfenfluramine);
gemischte Serotonin-/Dopamin-Wiederaufnahme-Inhibitoren (z.B. Bupropion) oder feste Kombinationen von Bupropion mit Naltrexon oder Bupropion mit Zonisamid;
gemischte Wiederaufnahmeinhibitoren wie z.B. DOV-21947;
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
gemischte Dopamin/Norepinephrin/Acetylcholin-Wiederaufnahme-Inhibitoren (z.B. Tesofensine) oder solchen wie sie z.B. in WO2006085118 beschrieben sind;
5-HT2C Rezeptor Agonisten (wie z.B. Lorcaserin Hydrochlorid (APD-356) oder BVT-933 oder solche, wie sie in WO200077010, WO200077001-02, WO2005019180, WO2003064423, WO200242304, WO2005035533, WO2005082859, WO2006004937, US2006025601, WO2006028961, WO2006077025, WO2006103511, WO2007028132, WO2007084622, US2007249709; WO2007132841, WO2007140213 beschrieben sind);
5-HT6 Rezeptor Modulatoren, wie z.B. E-6837, BVT-74316 oder PRX-07034 oder solche wie sie z.B. in WO2005058858, WO2007054257, WO2007107373, WO2007108569, WO2007108742-744 beschrieben sind;
Agonisten des Estrogenrezeptors gamma (ERR Agonisten), wie sie z.B. in WO2007131005 beschrieben sind;
Sigma-1 Rezeptorantagonisten, wie sie z.B. in WO2007098953, WO2007098961 beschrieben sind;
Muscarin 3 Rezeptor (M3R) Antagonisten, wie sie z.B. in WO2007110782 beschrieben sind;
Bombesin-Rezeptor Agonisten (BRS-3 Agonisten);
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO O1/85695));
Growth Hormone Secretagogue Receptor Antagonisten (Ghrelin Antagonisten) wie z. B. A-778193 oder solchen, wie sie in WO2005030734, WO2007127457 beschrieben sind;
Growth Hormone Secretagogue Receptor Modulatoren wie z.B. JMV-2959, JMV-3002, JMV-2810, JMV-2951 oder solchen, wie sie in WO2006012577 (z.B. YIL-781 oder YIL-870), WO2007079239 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
Dopaminagonisten (DA-Agonisten, z.B. Bromocriptin, Doprexin);
Lipase/Amylase-Inhibitoren (z.B. WO 00/40569);
Inhibitoren der Diacylglycerol O-Acyltransferasen (DGATs) wie z. B. BAY-74-4113 oder wie z. B. in US2004/0224997, WO2004094618, WO200058491, WO2005044250, WO2005072740, JP2005206492, WO2005013907, WO2006004200, WO2006019020, WO2006064189, WO2006082952, WO2006120125, WO2006113919, WO2006134317, WO2007016538, WO2007060140, JP2007131584, WO2007071966, WO2007126957, WO2007137103, WO2007137107, WO2007138304, WO2007138311 beschrieben;
Inhibitoren der Fettsäuresynthase (FAS) wie z.B. C75 oder solchen, wie in WO2004005277 beschrieben;
Inhibitoren der Stearoyl-CoA delta9 Desaturase (SCD1) wie sie z.B. in WO2007009236, WO2007044085, WO2007046867, WO2007046868, WO20070501124, WO2007056846, WO2007071023, WO2007130075, WO2007134457, WO2007136746 beschrieben sind;
Inhibitoren des "Adipocyte fatty acid-binding protein aP2" wie z.B. BMS-309403;
Aktivatoren der Adiponectinsekretion, wie z.B. in WO2006082978 beschrieben;
Promotoren der Adiponectinproduktion, wie z.B. in WO2007125946 beschrieben;
Oxyntomodulin;
Oleoyl-Estron
oder Agonisten oder partiellen Agonisten des Schilddrüsenhormonrezeptors (thyroid hormone receptor agonists) wie z. B: KB-2115 oder DITPA oder solche, wie in WO20058279, WO200172692, WO200194293, WO2003084915, WO2004018421, WO2005092316, WO2007003419, WO2007009913, WO2007039125, WO2007110225, WO2007110226, WO2007128492, WO2007132475, WO2007134864 beschrieben
oder Agonisten des Schilddrüsenhormonrezeptors beta (TR-beta) wie z. B. MB-07811 oder MB-07344, verabreicht.

Bei einer Ausführungsform der Erfindung wird die Verbindung der Formel I in Kombination mit einem Inhibitor der Site-1 Protease (S1P), wie z.B. PF-429242, verabreicht.

Bei einer weiteren Ausführungsform der Erfindung wird die Verbindung der Formel I in Kombination mit einem RNAi Therapeutikum, welches gegen PCSK9 (Proprotein Convertase Subtilisin/Kexin Typ 9) gerichtet ist, verabreicht.

Bei einer Ausführungsform wird die Verbindung der Formel I in Kombination mit Omacor® oder Lovaza™ (Omega-3-Fettsäureester; hochkonzentrierte Ethylester der Eicosapentaensäure und der Docosahexaensäure) verabreicht.

Bei einer Ausführungsform wird die Verbindung der Formel I in Kombination mit Lycopin verabreicht.

Bei einer Ausführungsform der Erfindung wird die Verbindung der Formel I in Kombination mit einem Antioxidans, wie z.B. OPC-14117, Succinobucol, Probucol, Tocopherol, Ascorbinsäure, ß-Caroten oder Selen verabreicht.

Bei einer Ausführungsform der Erfindung wird die Verbindung der Formel I in Kombination mit einem Vitamin, wie z. B. Vitamin B6 oder Vitamin B 12 verabreicht.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer anderen Ausführungsform wird die Verbindung der Formel I in Kombination mit einem Inhibitor der Carboanhydrase Typ 2 (Carbonic anhydrase type 2), wie z.B. solchen, wie in WO2007065948 beschrieben, verabreicht.

Bei einer anderen Ausführungsform wird die Verbindung der Formel I in Kombination mit Topiramat verabreicht.

Bei einer weiteren Ausführungsform wird die Verbindung der Formel I in Kombination mit einer festen Kombination von Topiramat mit Phentermin (Qnexa™) verabreicht.

Bei einer weiteren Ausführungsform wird die Verbindung der Formel I in Kombination mit einer Antisense-Verbindung, z.B. ISIS-377131, verabreicht, welche die Produktion des Glukokortikoidrezeptors inhibiert.

Bei einer Ausführungsform wird die Verbindung der Formel I in Kombination mit einem Agonisten des RUP3 Rezeptors, wie z. B. in WO2007035355 beschrieben, verabreicht.

Bei einer anderen Ausführungsform wird die Verbindung der Formel I in Kombination mit einem Aktivator des Gens, welches für die Ataxia Telangiectasia Mutated (ATM) Proteinkinase kodiert, wie z. B. Chloroquin, verabreicht.

Bei einer Ausführungsform wird die Verbindung der Formel I in Kombination mit einem Tau-Protein-Kinase-1-Inhibitor (TPK1 Inhibitor), wie z. B. in WO2007119463 beschrieben, verabreicht.

Bei einer Ausführungsform wird die Verbindung der Formel I in Kombination mit einem "c-Jun N-terminal kinase" Inhibitor (JNK-Inhibitor), wie z. B. in WO2007125405 beschrieben, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Modulatoren des Glukokortikoidrezeptors (GR), wie z.B. KB-3305 oder solchen Verbindungen wie sie z. B. in WO2005090336, WO2006071609, WO2006135826, WO2007105766 beschrieben sind, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIRT1 (einer NAD⁺-abhängigen Proteindeacetylase); dieser Wirkstoff kann z.B. Resveratrol in geeigneten Formulierungen sein, oder solche Verbindungen wie sie in WO2007019416 (z.B. SRT-1720) genannt sind.

Bei einer Ausführungsform der Erfindung ist der weitere Wirkstoff DM-71 (N-Acetyl-L-Cystein mit Bethanechol).

Bei einer Ausführungsform wird die Verbindung der Formel I in Kombination mit antihypercholesterolemisch wirkenden Verbindungen, wie sie z.B. in WO2007107587, WO2007111994 beschrieben sind, verabreicht.

Bei einer anderen Ausführungsform wird die Verbindung der Formel I in Kombination mit einem cyclischen Peptidagonisten des VPAC2 Rezeptors, wie sie z.B. in WO2007101146, WO2007133828 beschrieben sind, verabreicht.

Bei einer weiteren Ausführungsform wird die Verbindung der Formel I in Kombination mit einem Agonisten des Endothelinrezeptors, wie sie z.B. in WO2007112069 beschrieben sind, verabreicht.

Bei einer weiteren Ausführungsform wird die Verbindung der Formel I in Kombination mit AKP-020 (Bis(ethylmaltolato)oxovanadium-IV) verabreicht.

Bei einer anderen Ausführungsform wird die Verbindung der Formel I in Kombination mit gewebe-selektiven Androgenrezeptor Modulatoren ("tissue-selective androgen receptor modulators"; SARM), wie sie z.B. in WO2007099200 beschrieben sind, verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer anderen Ausführungsform der Erfindung ist der weitere WirkstoffMetreleptin (rekombinantes Methionyl-Leptin) kombiniert mit Pramlintide.

Bei einer weiteren Ausführungsform der Erfindung ist der weitere Wirkstoff das Tetrapeptid ISF-402.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer weiteren Ausführungsform ist der weitere Wirkstoff Geniposidinsäure (geniposidic acid; WO2007100104).

Bei einer Ausführungsform wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

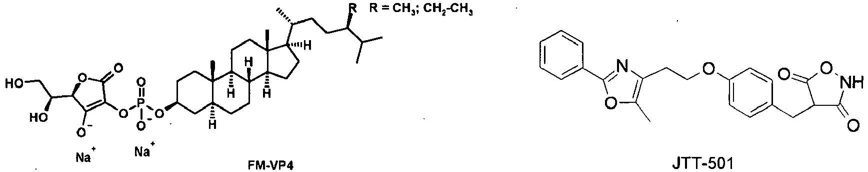

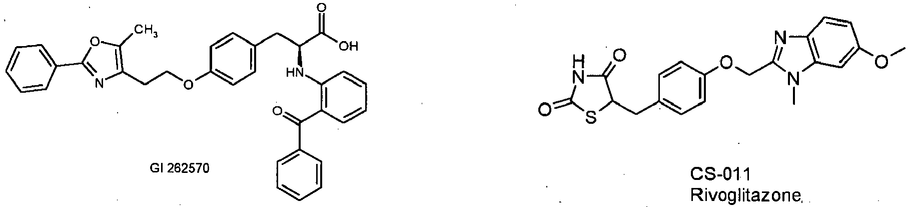

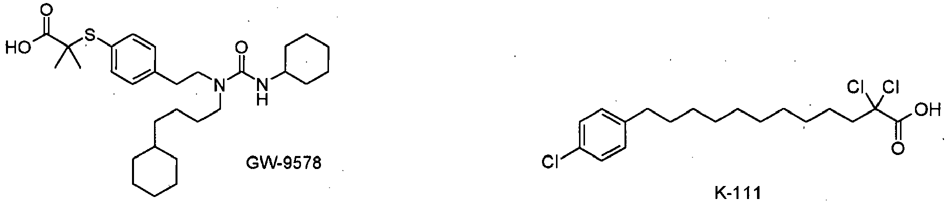

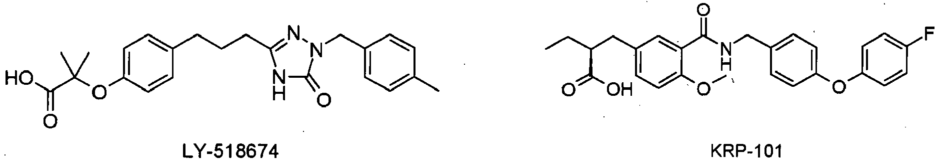

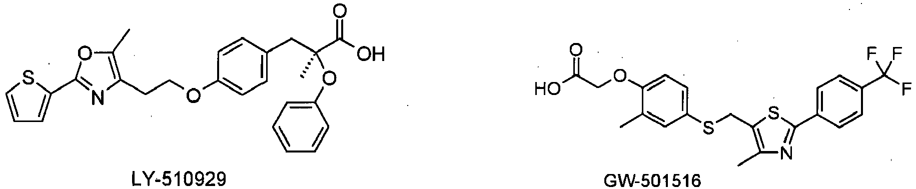

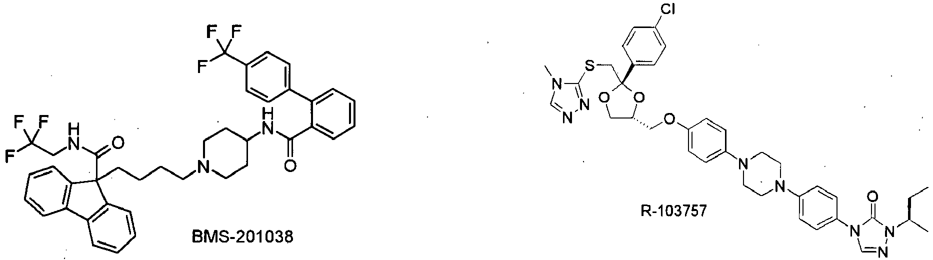

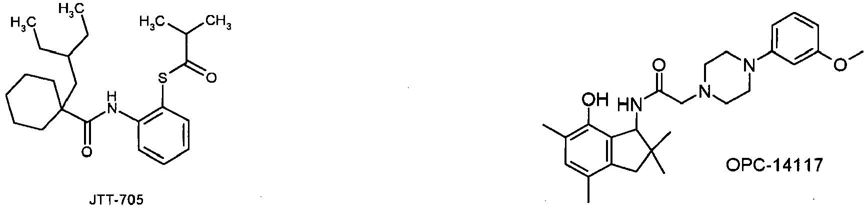

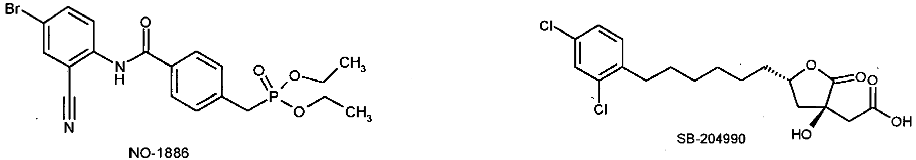

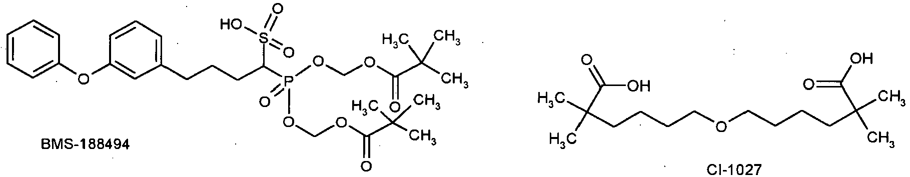

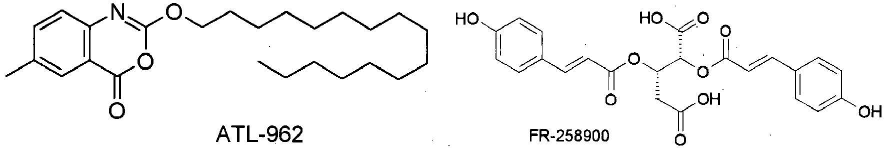

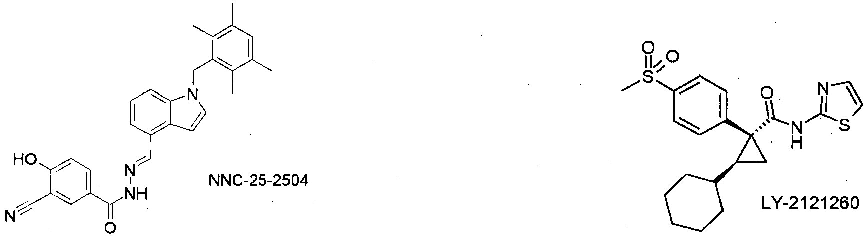

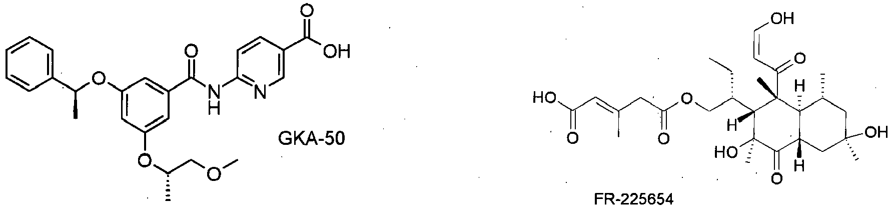

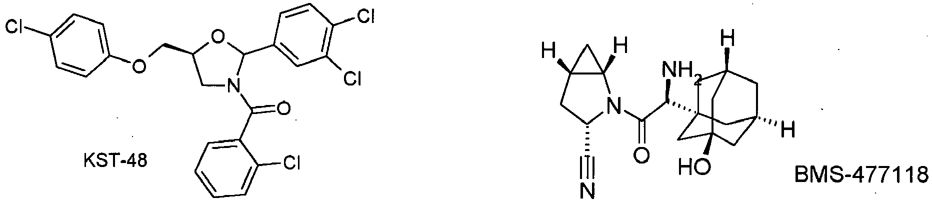

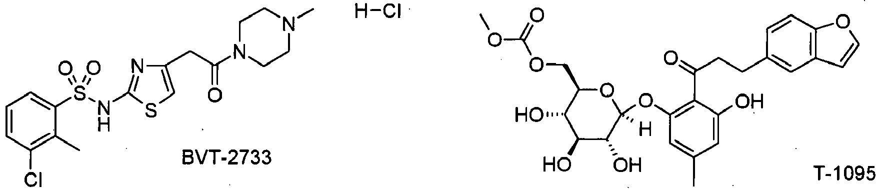

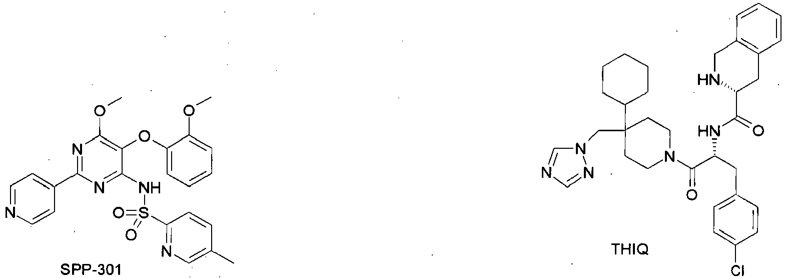

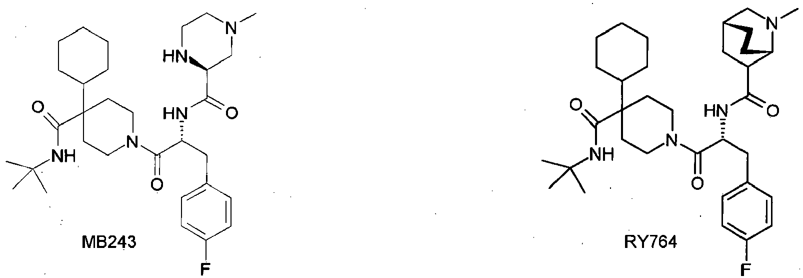

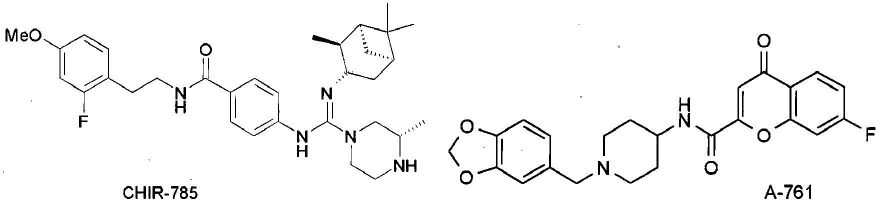

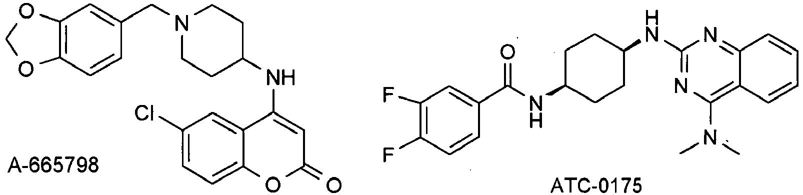

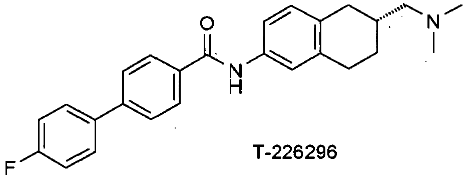

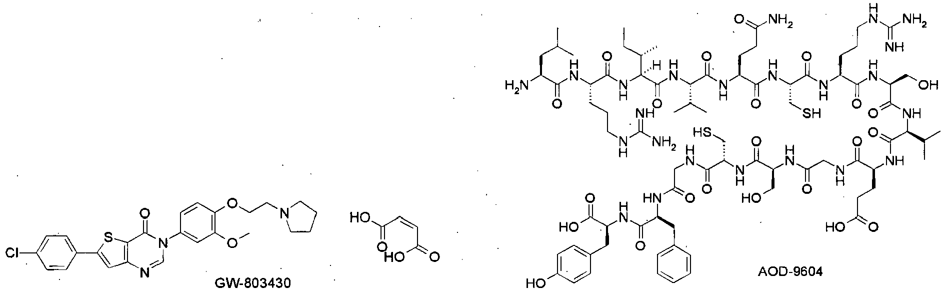

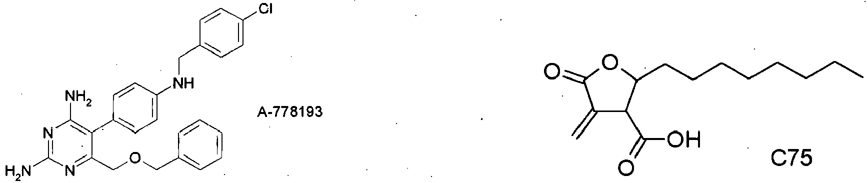

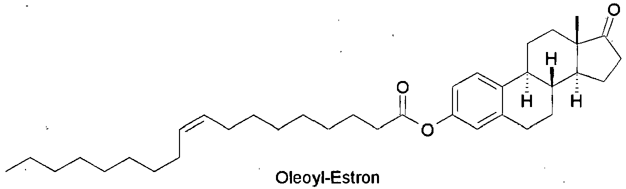

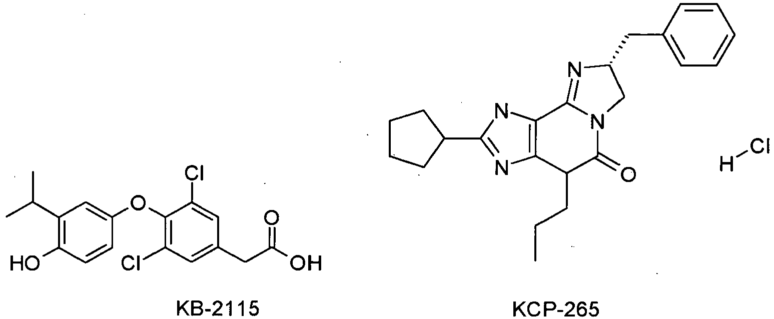

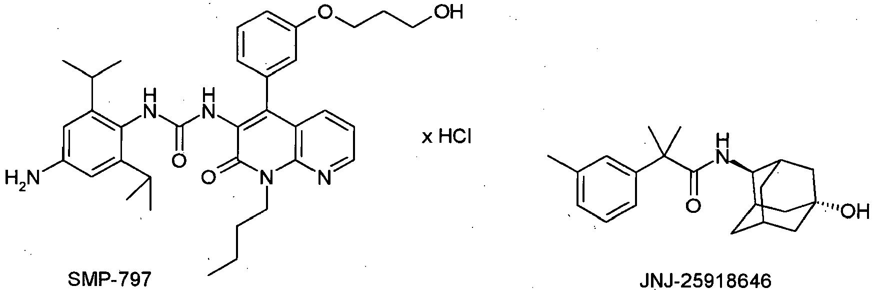

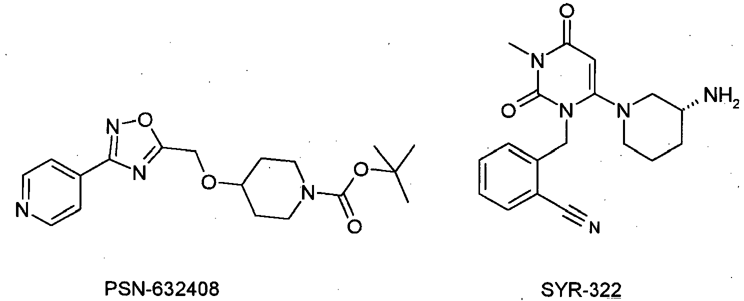

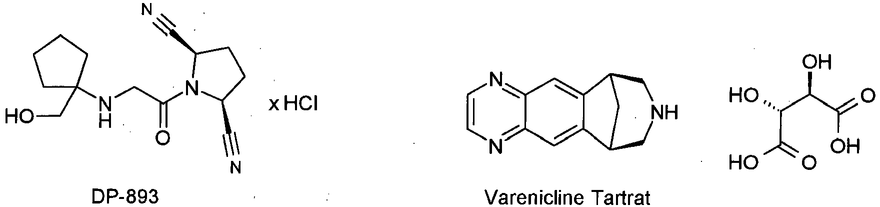

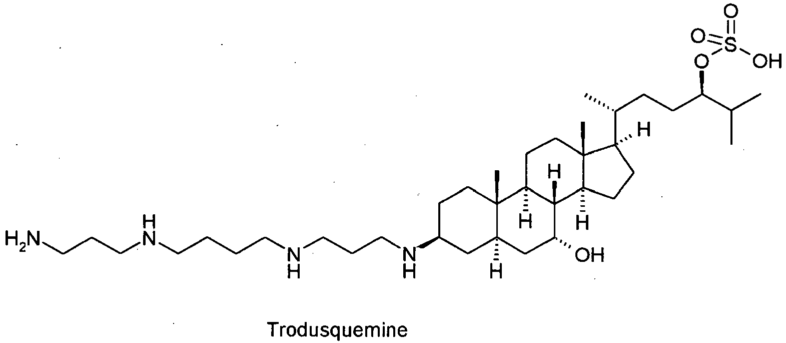

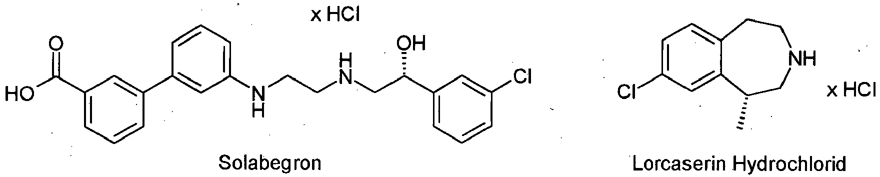

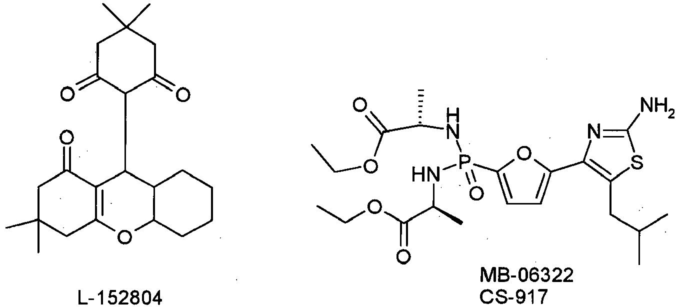

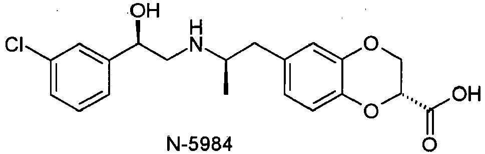

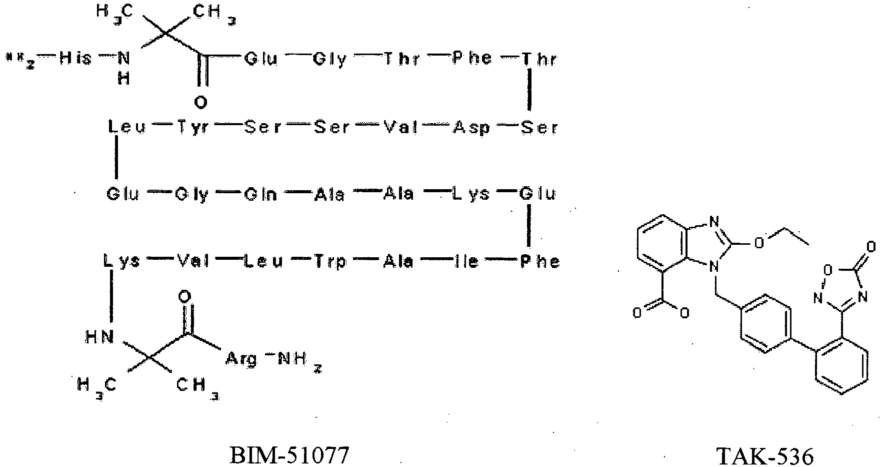

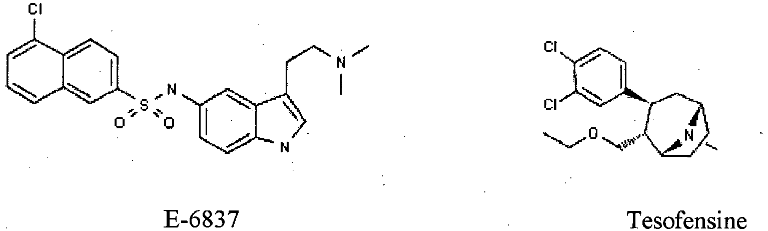

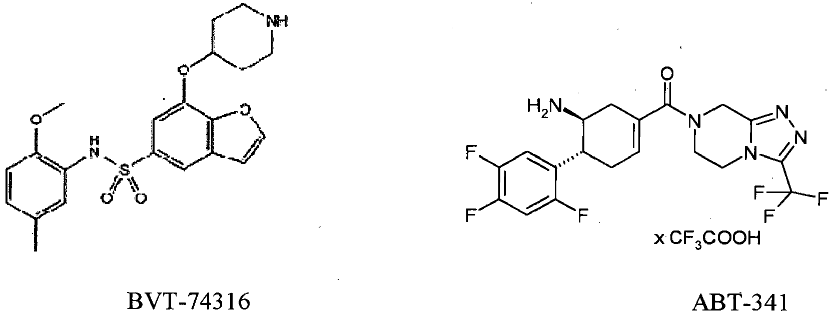

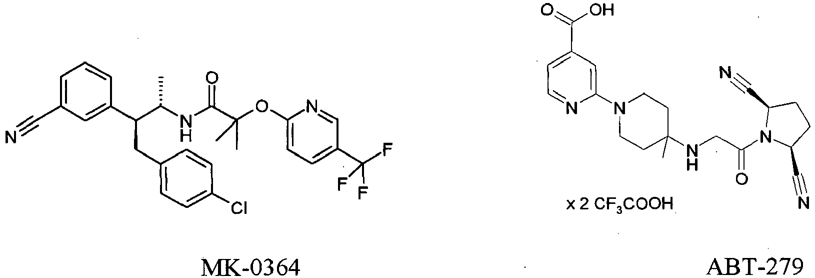

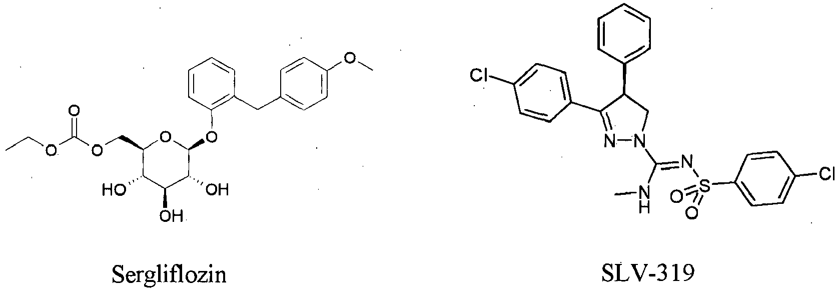

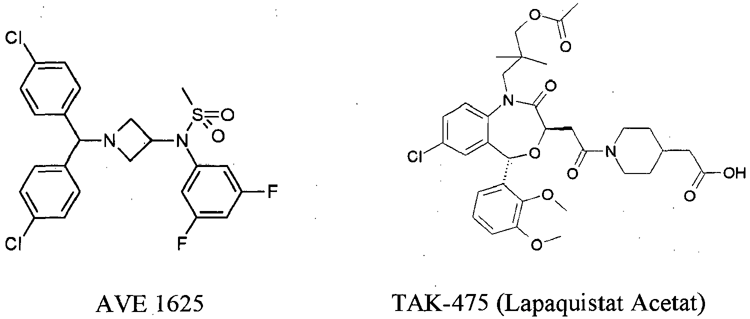

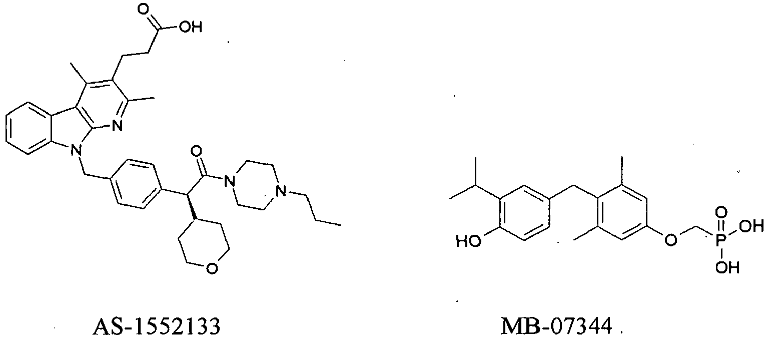

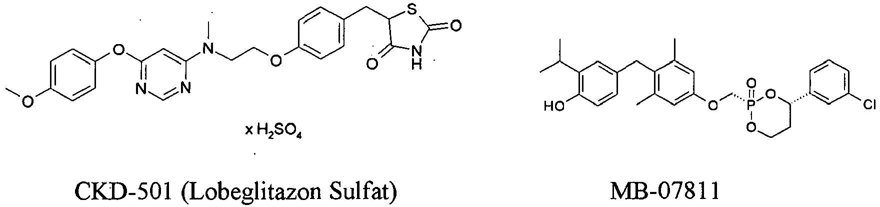

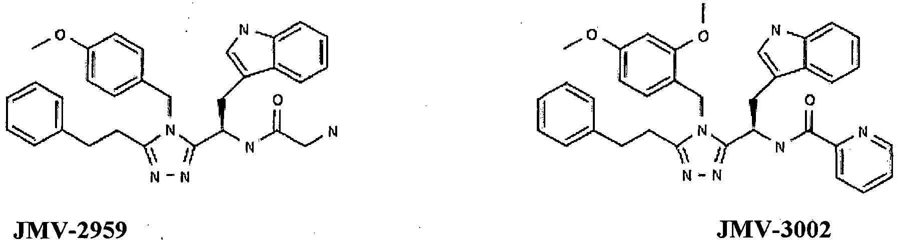

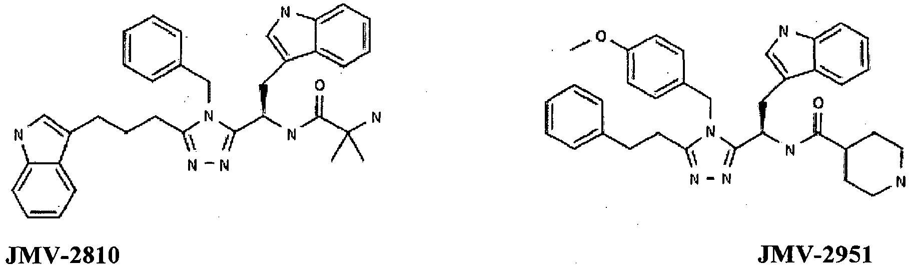

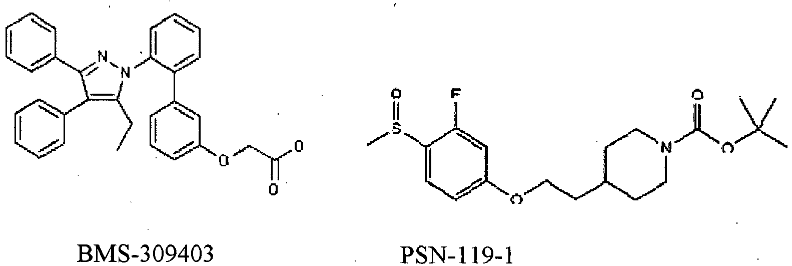

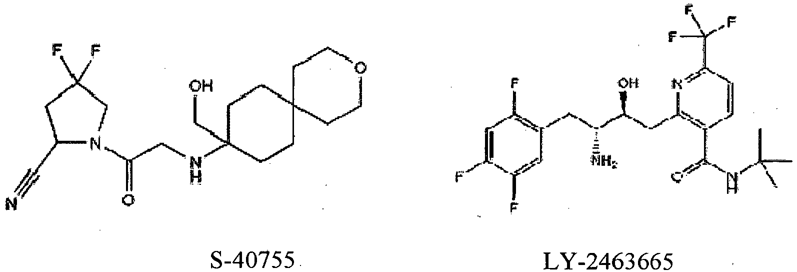

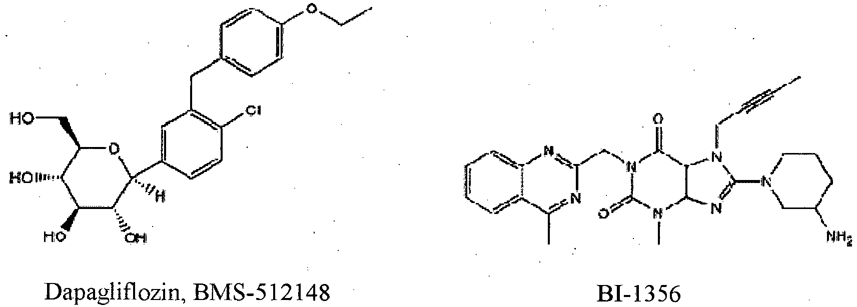

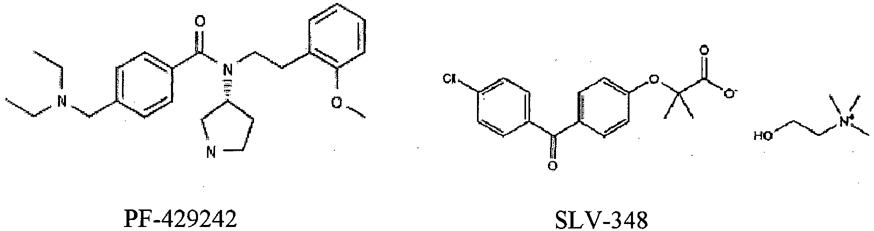

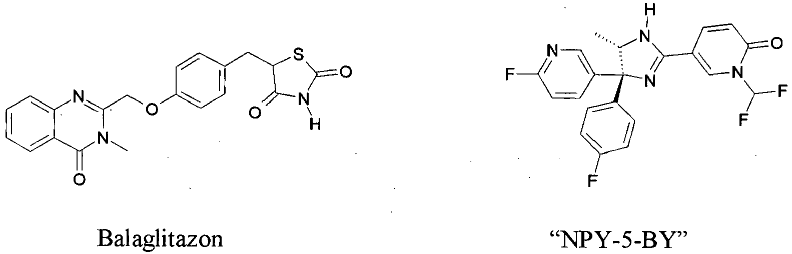

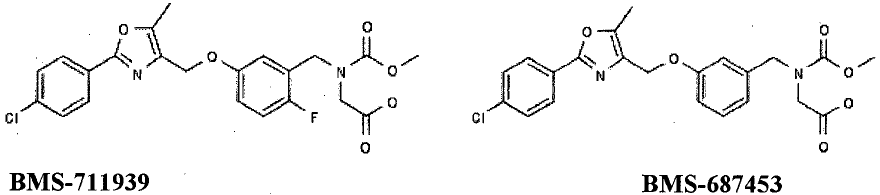

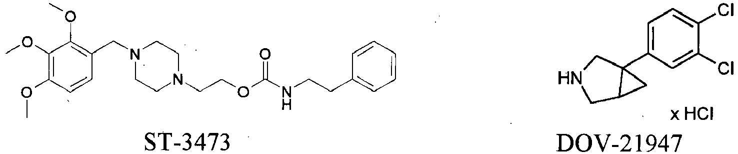

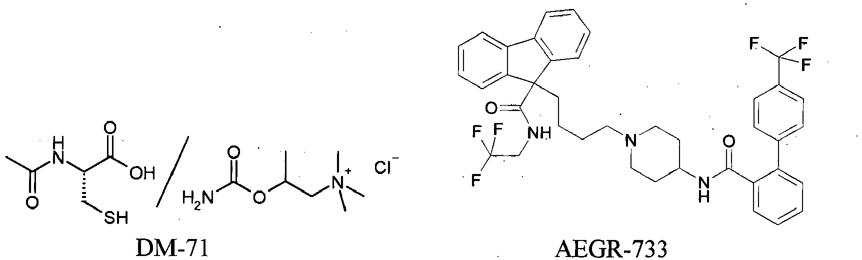

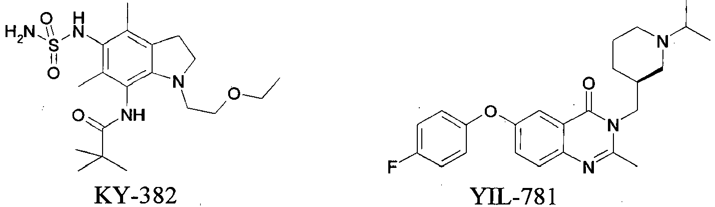

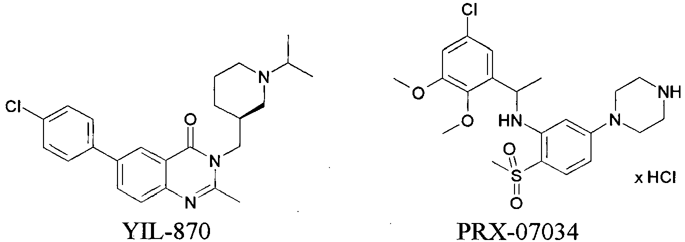

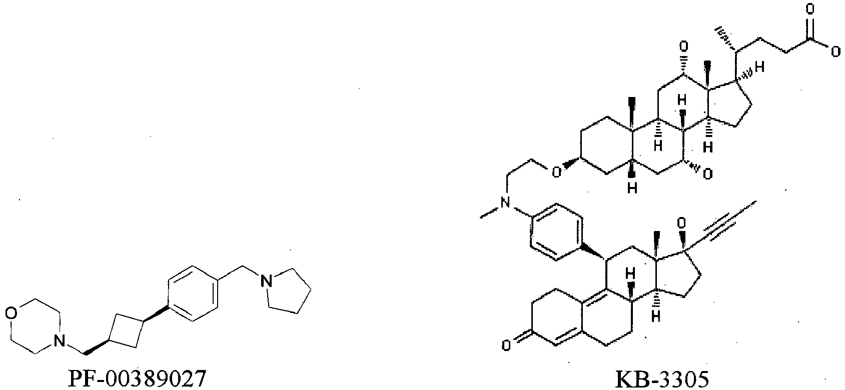

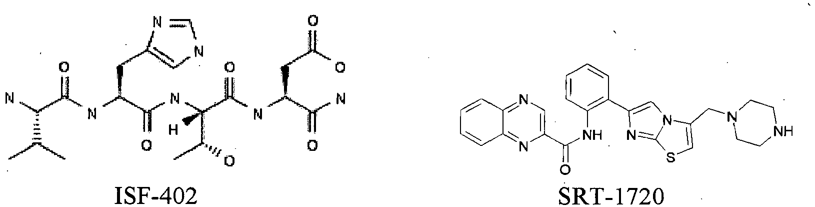

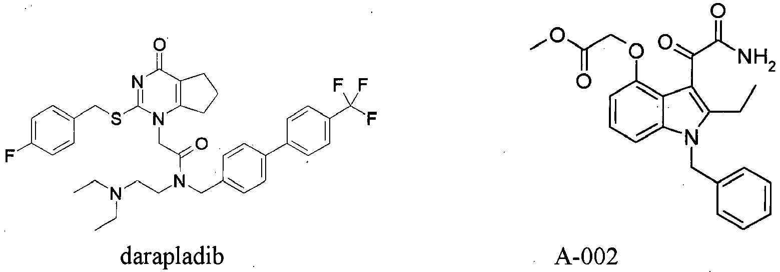

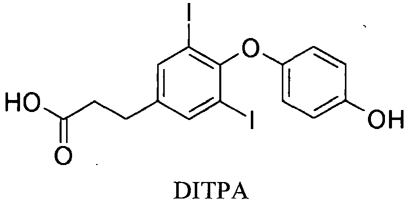

### Beispiele

Die nachfolgend aufgeführten Beispiele und Herstellungsmethoden dienen zur Erläuterung der Erfindung, ohne diese jedoch einzuschränken.

Die erfindungsgemäßen Verbindungen der Formel I können mit Hilfe von im Prinzip bekannten Reaktionen hergestellt werden. Beispielsweise wurden die Verbindungen nach folgenden allgemeinen Reaktionsschemata erhalten.

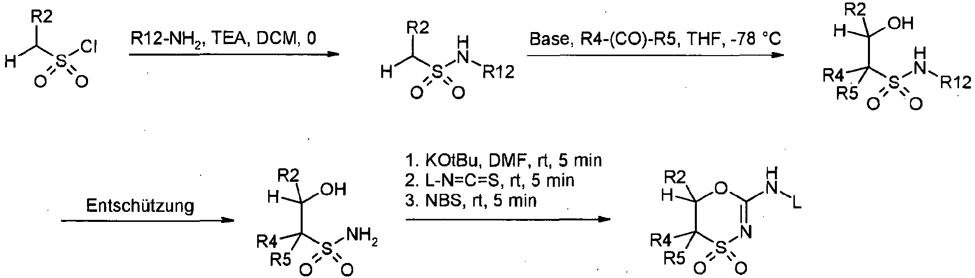

Aus einem mit R2 substituierten Methansulfonsäurechlorid wird durch die Reaktion mit einem geeigneten Amin und Triethylamin ein entsprechendes mit R2 substituiertes und mit R12 (z.B. Boc, Benzyl, 2,4-Dimethoxybenzyl) geschütztes Methansulfonsäureamid hergestellt. Durch Behandlung mit einer geeigneten Base (z.B. Methyllithium) kann bei tiefer Temperatur dann ein Dianion erzeugt werden, das mit einem Keton oder einem Aldehyd zum mit R12 geschützten Hydroxysulfonamid reagiert. Durch Entschützung von R12 (z.B. bei einer Boc-Gruppe oder einer 2,4-Dimethoxybenzylgruppe durch Säurebehandlung oder bei einer Benzylgruppe durch Hydrierung) entsteht das freie Hydroxysulfonamid. Die Behandlung des Hydroxysulfonamids mit Base und einem Isothiocyanat sowie nachfolgendem oxidativen Ringschluss mit NBS liefert die gewünschten 4,4-dioxo-oxathiazine.

In den Fällen, in denen funktionelle Gruppen (z.B. eine Hydroxylgruppe) in geschützter Form (z.B. benzylgeschützt oder als Ester) eingebracht werden, werden diese am Schluss der Synthese durch ein geeignetes Verfahren (z.B. Hydrierung oder Reduktion) freigesetzt.

Die verwendeten Isothiocyanate werden durch die Reaktion eines primären Amins mit Thiocarbonyldiimidazol erhalten, wobei eventuell vorhandene störende funktionelle Gruppen, z.B. Hydroxylgruppen mit geeigneten Schutzgruppen, z.B. Silylether blockiert werden. Die Schutzgruppen werden am Ende der Sequenz durch geeignete Verfahren entfernt, z.B. Silylgruppen durch Behandlung mit methanolischer Salzsäure.

In den Fällen, in denen während der Synthese Diastereomere oder Racemate entstehen, können diese durch präparative HPLC voneinander getrennt werden.

Einige der eingesetzten primären Amine sind kommerziell erhältlich.

4-Fluoro-bicyclo[2.2.2]octan-1-amin kann hergestellt werden wie in der Literatur beschrieben (JOC 1982, 47, 1952-7).

Andere erfindungsgemäße Verbindungen können auf weiteren Wegen erhalten werden, die im folgenden Schema beispielhaft skizziert sind.

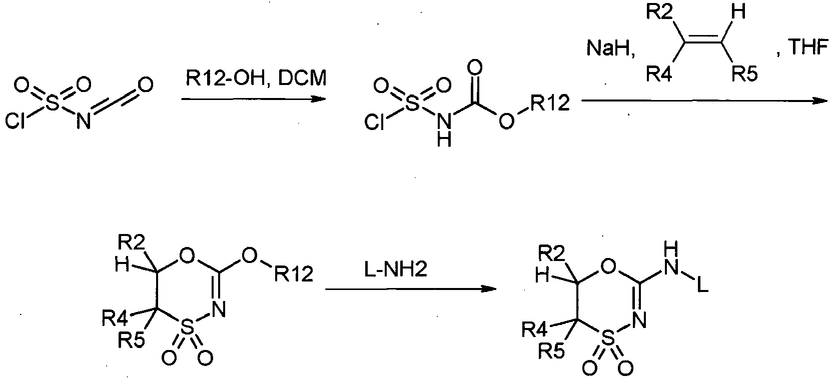

In Analogie zu einem literaturbekannten Verfahren (JACS 1972, 94, 4386-7) wird Chrosulfonylisocyanat mit einem Alkohol (z.B. Methanol) behandelt, wobei ein entsprechendes Carboalkoxysulfamoylchlorid entsteht (z.B. Carbomethoxysulfamoylchlorid). Dieses kann mit Natriumhydrid deprotoniert werden und die nach Chloridabspaltung entstehende Zwischenstufe (z.B. Methyl-N-Sulfonylurethan) reagiert in einer 2+4-Cycloaddition mit Alkenen unter Bildung eines alkoxysubstitierten (z.B. methoxysubstitierten) 4,4-Dioxooxathiazins. Die Alkoxygruppe lässt sich hier in einem geeigneten Lösemittel (z.B. Dichlormethan) durch ein Amin ersetzen, wobei die gewünschten 4,4-Dioxooxathiazine entstehen.

In den Fällen, in denen während der Synthese Diastereomere oder Racemate entstehen, können diese durch präparative HPLC voneinander getrennt werden.

In den Fällen, in denen funktionelle Gruppen (z.B. eine Hydroxylgruppe) in geschützter Form (z.B. benzylgeschützt oder als Ester) eingebracht werden, werden diese am Schluss der Synthese durch ein geeignetes Verfahren (z.B. Hydrierung oder Reduktion) freigesetzt.

Einige der verwendeten Amine sind kommerziell erhältlich oder können durch literaturbekannte Verfahren hergestellt werden.

Andere der verwendeten primären Amine wurden hergestellt wie im folgenden Schema beispielhaft skizziert ist.

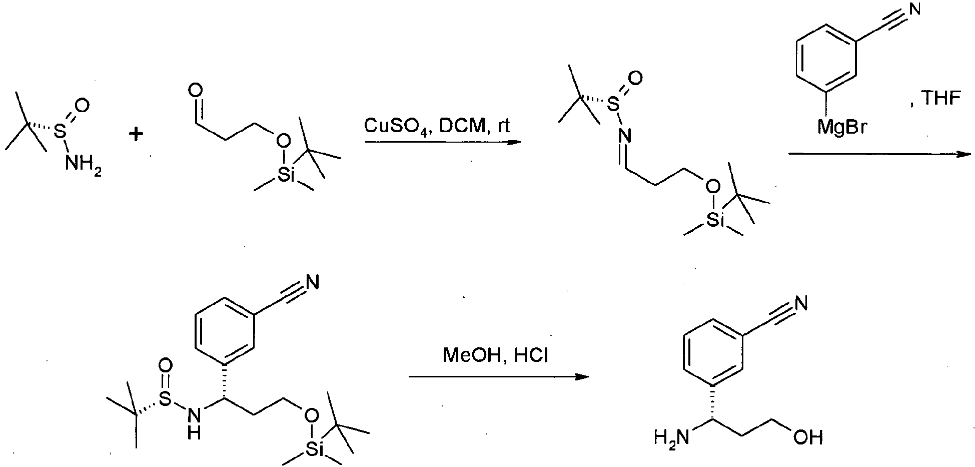

Die nachfolgend aufgeführten Beispiele dienen zur Erläuterung der Erfindung, ohne diese jedoch einzuschränken.

**Tabelle 1:**

| Beispiel | CHEMISTRY | Methode | Retentionszeist | Molgew icht (g/mol) | Name |
|---|---|---|---|---|---|
| 1 | 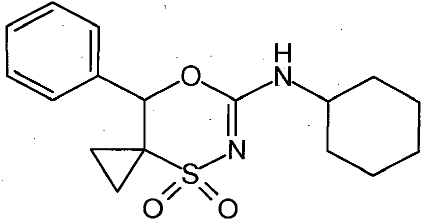 | A | 1.487 | 334.4 | Cyclohexyl-(4,4-dioxo-8-phenyl-7-oxa4lambda6-thia-5-aza-spiro[2.5]oct-5-en-6-yl)amin |
| 2 | 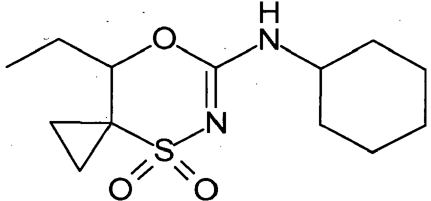 | A | 1.289 | 286.4 | Cyclohexyl-(8-ethyl-4,4-dioxo-7-oxa-4lambda6 thia-5-aza-spiro[2.5]oct-5-en-6-yl)-amin |
| 3 | 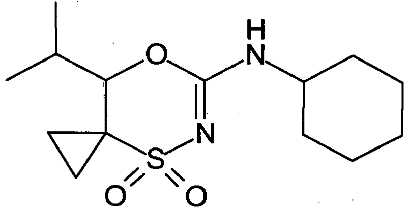 | A | 1.357 | 300.4 | Cyclohexyl-(8-isopropyl-4,4-dioxo-7-oxa-4lambda6-thia-5-aza-spiro[2.5]oct-5-en-6-yl)amin |
| 4 | 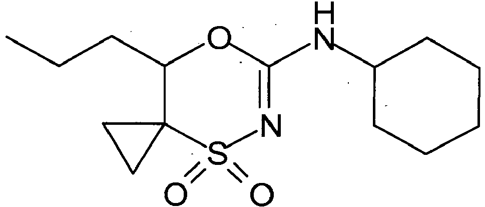 | A | 1.403 | 300.4 | Cyclohexyl-(4,4-dioxo-8-propyl-7-oxa4lambda6-thia-5-aza-spiro[2.5]oct-5-en-6-yl)-amin |
| 5 | 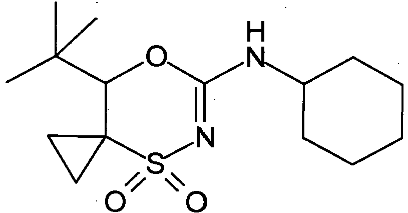 | A | 1.448 | 314.4 | (8-tert-Butyl-4,4-dioxo-7-oxa-4lambda6-thia-5aza-spiro[2.5]oct-5-en-6-yl)-cyclohexyl-amin |
| 6 | 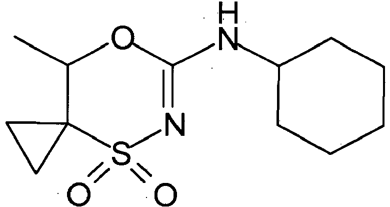 | A | 1.157 | 272.4 | Cyclohexyl-(8-methyl-4,4-dioxo-7-oxa4lambda6-thia-5-aza-spiro[2.5]oct-5-en-6-yl)-amin |

### Chromatographie-Methoden:

### Methode A

Säule: YMC J'spere ODS H80, 80 Ǻ, S-4 µm, 20 x 2.1 mm
Laufmittel: 0 min 96 % H₂O (0.05 % TFA) - 2.0 min 95 % Acetonitril - 2.4 min 95 % Acetonitril - 2.45 min 4% Acetonitril (30 °C, Fluss 1 ml / min)

### Methode B

Säule: Mercury MS, Luna C18(2), S-3 µm, 10 x 2.0 mm
Laufmittel: 0 min 93 % H₂O (0.05 % TFA) - 1.2 min 95 % Acetonitril - 1.4 min 95 % Acetonitril - 1.45 min 7% Acetonitril (30 °C, Fluss 1.1 ml / min)

### Methode C

Säule: Mercury MS, Luna C18(2), S-3 µm, 10 x 2,0 mm
Laufmittel: 0 min 93 % H₂O (0.05 % TFA) - 1.0 min 95 % Acetonitril - 1.45 min 95 % Acetonitril - 1.5 min 7% Acetonitril (30 °C, Fluss 1.1 ml / min)

Die Wirksamkeit der Verbindungen wurde wie folgt getestet:

### Enzymatischer 11beta-HSD1 Test:

Zum Messen der Wirkung der Verbindungen wurde ein auf SPA basierendes Nachweisverfahren (Solly *et al.* 2005) angewendet. Zunächst wurden 20 µl der humanen 11β-HSD1-Mikrosomenfraktion (0,2 µg Protein), zubereitet in 50 mM HEPES, 0,1% BSA (w/v), in eine Platte mit 384 Vertiefungen gegeben. Die Testverbindungen (0,09 µl) wurden in 100% DMSO auf die Assayplatte übertragen. Die Reaktion wurde durch Zugabe von 20 µl [1,2-³H] Cortison (0,1 µCi/100 mM) in Assaypuffer mit 25 mM HEPES, 100 mM KCl, 5 mM NaCl, 2 mM MgCl₂ und 0,25 mM NADPH gestartet. Die Platte wurde 1 Stunde lang bei 37°C geschüttelt. Gleichzeitig wurde eine Stopplösung mit 20 mg/ml SPA-PVT-Perlen, 1,6 mg/ml monoklonalem Cortisol-Antikörper und 0,01 mM SSR110887 (Inhibitor aus dem Biovitrium-Patent) in 50 mM HEPES, 1 M NaCl und 1 M KCl bei Raumtemperatur gerührt. Zum Abbruch der Reaktion wurden in alle Vertiefungen jeweils 25 µl der Stopplösung gegeben. Die Platte wurde 1 weitere Stunde lang sachte bei Raumtemperatur geschüttelt und dann 1 min bei 500 gₐᵥ zentrifugiert, damit sich die SPA-Perlen absetzen konnten. Die Platte wurde dann in einem Wallac-1450-Microbeta-Gerät mit einem Standard-SPA-Programm gelesen (1 min Zählzeit/Vertiefung). Die Vergleichsverbindung war Glycyrrhetinsäure.

Protein und radioaktives Substrat wurden mit einem Biomek FX-Gerät (Beckman Coulter) zur Handhabung von Flüssigkeiten dispensiert. Die Testverbindungen wurden mit einem mit einem 90-nl-Pin-Tool ausgestatteten Cybi-Well (CyBio) zugesetzt.

Lit.: Solly S, Mundt SS, Zokian HJ, Juy-Fang Ding G, Hermanowski-Vosatka A, Strulovici B and Zheng W. High-throughput screening of 11β-Hydroxysteroid dehydrogenase type 1 in scintillation proximity format. Assay Drug Dev Technol 2005;3:377-384.

**Tabelle 2: Biologische Aktivität in Nanomolar (nM)**

| Beispiel | IC₅₀ (nM) |
|---|---|
| 1 | 355 |
| 2 | 177 |
| 3 | 629 |
| 4 | 518 |
| 5 | 438 |
| 6 | 598 |

Aus den Messdaten ist abzulesen, dass die Verbindungen der Formel I 11beta-HSD1 (11beta-Hydroxysteroid Dehydrogenase Type 1) inhibieren und dadurch gut zur Behandlung von Hyperglykämie, Insulin Resistenz, Diabetes, Adipositas, Lipidstoffwechselstörungen, Bluthochdruck, Verbesserung der Kognition, erhöhtem Augeninnendruck, der Förderung der Wundheilung und anderen Erkrankungen geeignet sind.

Nachfolgend wird die Herstellung einiger Beispiele detailliert beschrieben, die übrigen Verbindungen der Formel I wurden analog erhalten:

### Experimenteller Teil:

### Cyclohexyl-(4,4-dioxo-8-phenyl-7-oxa-4lambda*6*-thia-5-aza-spiro[2.5]oct-5-en-6-yl)-amin

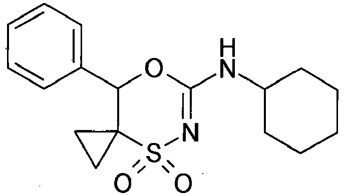

### Cyclopropanesulfonsäureamid

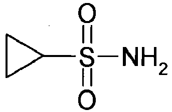

Es wurde ein Gemisch aus 50 ml 25 %iger wässriger Ammoniak-Lösung und 50 ml THF vorgelegt und anschließend langsam 10.00 g Cyclopropansulfonylchlorid in 10 ml THF tropfenweise zugegeben und 16 Stunden gerührt. Nach dem Einrotieren und Koevaporieren mit Toluol wurde der Rückstand mit 100 ml Ethylacetat ausgerührt und vom Feststoff filtriert. Die organische Phase wurde über Na₂SO₄ getrocknet und einrotiert. Man erhielt so das Produkt (7.03 g) mit dem Molekulargewicht 121.2 g / mol (C₃H₇NO₂S); MS (ESI): m / e = 122 (M+H⁺).

### N-tert-Butyloxycarbonyl-cyclopropansulfonsäureamid

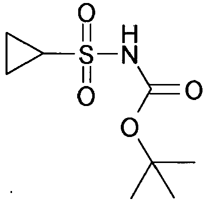

Es wurden 1.46 g Cyclopropansulfonsäureamid in einer Mischung aus 30 ml Dichlormethan und 1.67 ml Triethylamin vorgelegt und anschließend 2.63 g Di-tert-butyldicarbonat und 0.15 g 4-Dimethylaminopyridin zugegeben und 16 Stunden gerührt. Die Reaktionslösung wurde mit 1 N wässriger Salzsäure, gesättigter wässriger Natriumchlorid-Lösung und Wasser gewaschen, die organische Phase über Na₂SO₄ getrocknet und einrotiert. Man erhielt so das Produkt (2.66 g) mit dem Molekulargewicht 221.3 g / mol (C₈H₁₅NO₄S); MS (ESI): m / e = 166 (M-tert-Butyl+H⁺).

### N-tert-Butyloxycarbonyl-1-(hydroxy-phenyl-methyl)-cyclopropansulfonsäureamid

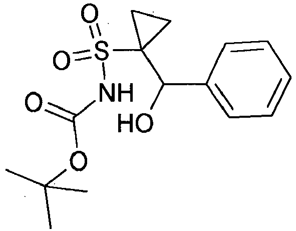

Unter Inertgas wurde 1.00 g N-tert-Butyloxycarbonyl-cyclopropansulfonsäureamid in 40 ml THF vorgelegt und anschließend bei einer Temperatur von -78 °C 6.63 ml einer 1.5 N Butyllithium-Lösung in Hexan tropfenweise zugegeben und 1 Stunde unter Eiskühlung gerührt. Nach weiteren 30 Minuten Rühren bei Raumtemperatur wurde erneut auf-78 °C abgekühlt und eine Lösung von 0.92 ml Benzaldehyd in 4 ml THF zugegeben. Die Mischung wurde über Nacht unter Rühren auf Raumtemperatur kommen gelassen. Die Reaktionslösung wurde mit 20 ml einer gesättigten wässrigen Ammoniumchlorid-Lösung verdünnt und mit 40 ml Ethylacetat extrahiert, die organische Phase über Na₂SO₄ getrocknet und einrotiert. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (880 mg) mit dem Molekulargewicht 327.4 g / mol (C₁₅H₂₁NO₅S); MS (ESI): m / e = 254 (M-tert-Butanol+H⁺).

### 1-(Hydroxy-phenyl-methyl)-cyclopropansulfonsäureamid

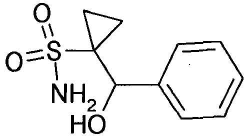

Es wurden 880 mg N-tert-Butyloxycarbonyl-1-(hydroxy-phenyl-methyl)-cyclopropansulfonsäureamid in 25 ml Ethylacetat gelöst und mit 13.4 ml einer 1 N wässrigen Salzsäure versetzt. Die Reaktionslösung wurde auf 65° C erhitzt und die Vollständigkeit der Umsetzung mit LCMS geprüft. Nach 8 Stunden wurde die Mischung mit gesättigter wässriger Natriumcarbonat-Lösung neutralisiert, die organische Phase über Na₂SO₄ getrocknet und einrotiert. Man erhielt so das Produkt (610 mg) mit dem Molekulargewicht 227.3 g / mol (C₁₀H₁₃NO₃S); MS (ESI): m / e = 210 (M-H₂O+H⁺).

### Cyclohexyl-(4,4-dioxo-8-phenyl-7-oxa-4lambda*6*-thia-5-aza-spiro[2.5]oct-5-en-6-yl)-amin

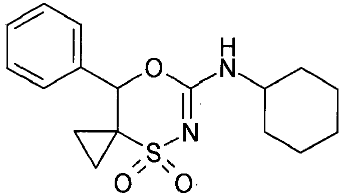

610 mg 1-(Hydroxy-phenyl-methyl)-cyclopropansulfonsäureamid wurden in 5 ml NMP gelöst und anschließend mit 331 mg Kalium-tert.-butylat für 5 Minuten gerührt. Dann wurden 569 mg Cyclohexylisothiocyanat zugegeben. Nach 15 Minuten rühren wurden dann 525 mg N-Bromsuccinimid zugesetzt und weitere 90 Minuten gerührt. Die Lösung wurde mit präparativer HPLC gereinigt. Man erhielt so das Produkt (191 mg) mit dem Molekulargewicht 334.4 g / mol (C₁₇H₂₂N₂O₃S); MS (ESI): m / e = 335 (M+H⁺).

Die Verbindungen 1,3-6, 8,9,17,18,20 und 40 wurden mit dieser Herstellungsvorschrift synthetisiert:
Cyclohexyl-(8,8-dimethyl-4,4-dioxo-7-oxa-4lambda6-thia-5-aza-spiro[2.5]oct-5-en-6-yl)-amin
Cyclohexyl-(8-ethyl-4,4-dioxo-7-oxa-4lambda6-thia-5-aza-spiro[2.5]oct-5-en-6-yl)-amin
Cyclohexyl-(8-isopropyl-4,4-dioxo-7-oxa-4lambda6-thia-5-aza-spiro[2.5]oct-5-en-6-yl)-amin
Cyclohexyl-(4,4-dioxo-8-propyl-7-oxa-4lambda6-thia-5-aza-spiro[2.5]oct-5-en-6-yl)-amin
(8-tert-Butyl-4,4-dioxo-7-oxa-4lambda6-thia-5-aza-spiro[2.5]oct-5-en-6-yl)-cyclohexylamin
Cyclohexyl-(8-methyl-4,4-dioxo-7-oxa-4lambda6-thia-5-aza-spiro[2.5]oct-5-en-6-yl)-amin
Cyclohexyl-(5,6,6-trimethyl-4,4-dioxo-5,6-dihydro-4H-4lambda6-[1,4,3]oxathiazin-2-yl)-amin
Cyclohexyl-(11,11-dioxo-6,8-dioxa-11lambda6-thia-10-aza-dispiro[2.0.3.4]undec-9-en-9-yl)-amin
Cyclohexyl-(8-methoxymethyl-8-methyl-4,4-dioxo-7-oxa-4lambda6-thia-5-aza-spiro[2.5]oct-5-en-6-yl)-amin
(8-Benzyloxymethyl-8-methyl-4,4-dioxo-7-oxa-4lambda6-thia-5-aza-spiro[2.5]oct-5-en-6-yl)-cyclohexyl-amin
Cyclohexyl-(11,11-dioxo-8-oxa-11lambda6-thia-10-aza-dispiro[2.0.3.4]undec-9-en-9-yl)-amin

### (6-Cyclohexylamino-8-methyl-4,4-dioxo-7-oxa-4lambda6-thia-5-aza-spiro[2.5]oct-5-en-8-yl)-methanol

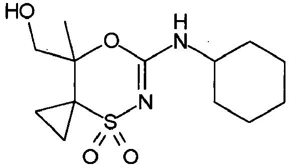

Unter Inertgas wurden 80 mg (8-Benzyloxymethyl-8-methyl-4,4-dioxo-7-oxa-4lambda6-thia-5-aza-spiro[2.5]oct-5-en-6-yl)-cyclohexyl-amin in 6 ml Ethanol vorgelegt und unter Zusatz von 5 mg Pd/C 5 % für 16 Stunden unter Wasserstoff-Atmosphäre gerührt. Die Vollständigkeit der Umsetzung wurde mit LCMS geprüft und die Reaktion nochmals mit 5 mg Pd/C 5 % versetzt und weitere 2 Stunden unter Wasserstoff-Atmosphäre gerührt. Nach Filtration von den festen Rückständen wurde das Filtrat unter vermindertem Druck vom Lösungsmittel befreit. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert, anschließend noch einmal in 10 ml einer Mischung aus Acetonitril und Wasser (9:1) aufgenommen und lyphilisiert. Man erhielt so das Produkt (32 mg) mit dem Molekulargewicht 302.4 g / mol (C₁₃H₂₂N₂O₄S), MS (ESI): m/e = 303 (M+H⁺).

### Cyclohexyl-(8-methyl-4,4-dioxo-8-trifluormethyl-7-oxa-4lambda6-thia-5-aza-spiro[2.5]oct-5-en-6-yl)-amin

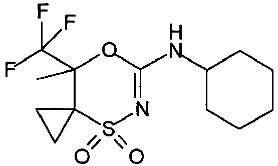

Die Darstellung erfolgte analog der oben beschriebenen Synthese der Verbindung 2. Die Einführung der zusätzlichen Methylgruppe wird nachfolgend beschrieben.

### 1-(2,2,2-Trifluor-1-hydroxy-1-methyl-ethyl)-cyclopropansulfonsäureamid

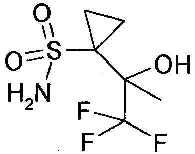

Unter Inertgas wurden 250 mg 1-(2,2,2-Trifluor-acetyl)-cyclopropansulfonsäureamid in 6 ml THF vorgelegt und anschließend bei einer Temperatur von -78 °C 1.15 ml einer 3.0 N Methylmagnesiumbromid-Lösung in Dieethylether tropfenweise zugegeben und 4 Stunden bei gleichbleibender Temperatur gerührt. Die Reaktionslösung wurde mit 5 ml einer gesättigten wässrigen Ammoniumchlorid-Lösung versetzt und unter Rühren auf Raumtemperatur kommen gelassen. Nach Einstellen mit 2 N wässriger Salzsäure auf pH5 wurde die Mischung mit 10 ml Ethylacetat extrahiert, die organische Phase über Na₂SO₄ getrocknet und einrotiert. Man erhielt so das Produkt (208 mg) mit dem Molekulargewicht 233.2 g / mol (C₆H₁₀F₃NO₃S); MS (ESI): m / e = 234 (M+H⁺).

### (8,8-Dimethyl-4,4-dioxo-7-oxa-4lambda6-thia-5-aza-spiro[2.5]oct-5-en-6-yl)-(4-fluorbicyclo[2.2.2]oct-1-yl)-amin

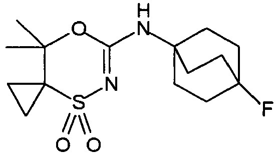

Das verwendete Amin wurde wie folgt hergestellt:

### Amino-4-fluor-bicyclo[2.2.2]otcan

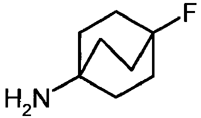

Journal of Organic Chemistry 1982, 47(15), 2951-2957 .

Es wurden 157 mg 4-Fluor-bicyclo[2.2.2]oct-1-ylamin Hydrochlorid in 2 ml Dichlormethan vorgelegt und anschließend 174 mg 1,1'-Thiocarbonyldiimidazol und 0.172 ml Triethylamin zugegeben. Nach 30 Minuten Rühren bei Raumtemperatur wurde der Ansatz mit einer Mischung aus 10 ml Diethylether und 10 ml n-Pentan versetzt und mit Wasser gewaschen. Die organische Phase wurde über MgSO₄ getrocknet, einrotiert und der Rückstand in 1.5 ml NMP gelöst. Diese Lösung wurde zu einer Suspension aus 146 mg 1-(1-Hydroxy-1-methyl-ethyl)-cyclopropansulfonsäureamid und 101 mg Kalium-tert-butylat in 1.5 ml NMP gegeben. Nach 1 Stunde Rühren wurden 149 mg N-Bromsuccinimid zugesetzt und die resultierende Reaktionslösung erneut 1 Stunde gerührt. Das Reaktionsgemisch wurde mit 40 ml Wasser versetzt und 3 x mit 15 ml Ethylacetat extrahiert. Die vereinten organischen Phasen wurden mit 1 N wässriger Kaliumhydrogensulfat-Lösung und zweimal mit gesättigter wässriger Natriumchlorid-Lösung gewaschen, über MgSO₄ getrocknet und einrotiert. Der Rückstand wurde im Reinigungslabor über präparative HPLC gereinigt und die produkthaltigen Fraktionen lyophilisiert. Man erhielt so das Produkt (9.1 mg) mit dem Molekulargewicht 330.4 g / mol (C₁₅H₂₃FN₂O₃S); MS (ESI): m / e = 331 (M+H⁺).

### (S)-3-(8,8-Dimethyl-4,4-dioxo-7-oxa-4lambda6-thia-5-aza-spiro[2.5]oct-5-en-6-ylamino)-3-phenyl-propan-1-ol

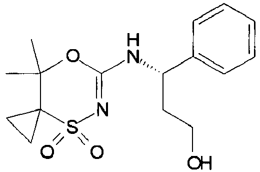

Es wurden 232 mg (S)-3-Amino-3-phenylpropan-1-ol in 5 ml Dichlormethan vorgelegt und anschließend 0.58 ml Triethylamin und 231 mg tert-Butyldimethylchlorsilan zugegeben. Die Reaktionslösung wurde bei Raumtemperatur gerührt und der Fortschritt der Umsetzung mit LCMS überprüft. Nach 1 Stunde wurden weitere 42 mg tert-Butyldimethylchlorsilan zugegeben und der Ansatz für 64 Stunden gerührt. Anschließend wurde die Reaktionslösung auf 10 ml Wasser gegeben und mit einer Mischung aus 10 ml Diethylether und 10 ml n-Pentan extrahiert. Nach dem Trocknen über Na₂SO₄ und Einrotieren wurde der Rückstand in 5 ml Dichlormethan gelöst und mit 274 mg 1,1'-Thiocarbonyldiimidazol versetzt und 30 Minuten gerührt. Die Reaktionslösung wurde 2 x mit 5 ml Wasser gewaschen, über Na₂SO₄ getrocknet, einrotiert und der Rückstand in 2 ml NMP gelöst.

250 mg 1-(1-Hydroxy-1-methyl-ethyl)-cyclopropansulfonsäureamid wurden in 2 ml NMP gelöst, unter Eiskühlung mit 0.70 ml einer 2 N Lösung von Natrium-bis(trimethylsilyl)-amid in THF und nach 5 Minuten Rühren mit der oben hergestelleten Isothiocyanat-Lösung versetzt. Nach 1.5 Stunden Rühren wurden 248 mg N-Bromsuccinimid zugesetzt und die resultierende Reaktionslösung 10 Minuten gerührt. Das Reaktionsgemisch wurde auf 20 ml Wasser gegeben und 3 x mit 20 ml Ethylacetat extrahiert. Die vereinten organischen Phasen wurden mit gesättigter wässriger Natriumchlorid-Lösung gewaschen, über Na₂SO₄ getrocknet und einrotiert. Der Rückstand wurde in 5 ml Methanol gelöst, mit 0.20 ml konzentrierter Salzsäure versetzt und für 1.5 Stunden gerührt. Nach der Zugabe von 1.26 ml 1 N wässriger Natronlauge wurde der Ansatz einrotiert und im Reinigungslabor über präparative HPLC gereinigt. Nach Lyophilisation der produkthaltigen Fraktionen erhielt man so das Produkt (26 mg) mit dem Molekulargewicht 338.4 g / mol (C₁₆H₂₂N₂O₄S), MS (ESI): m / e = 339 (M+H⁺).

### Cyclohexyl-(8,8-dioxo-5-oxa-8lambda6-thia-7-aza-spiro[3.5]non-6-en-6-yl)-amin

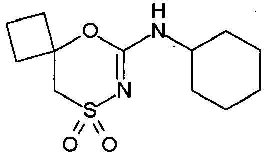

### N-Benzyl-methansulfonamid

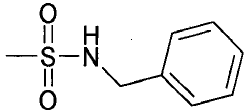

18.27 g Benzylamin wurden in 100 ml Methylenchlorid gelöst und unter Eiskühlung tropfenweise sehr langsam mit 6.00 ml Methansulfonylchlorid versetzt. Nach vollständiger Zugabe des Methansulfonylchlorids wurde die Ansatzlösung mit 100 ml Wasser verdünnt und die wässrige Phase erneut mit 50 ml Methylenchlorid extrahiert. Die vereinten organischen Phasen wurden mit 1 N wässriger Salzsäure gewaschen, über MgSO₄ getrocknet und einrotiert. Man erhielt so das Produkt (14.40 g) mit dem Molekulargewicht 185.3 g / mol (C₈H₁₁NO₂S).

### (1-Hydroxy-cyclobutyl)-methansulfonamid

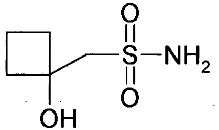

Unter Inertgas wurden 2.00 g N-Benzyl-methansulfonamid in 20 ml THF vorgelegt, anschließend bei einer Temperatur von -78 °C 13.50 ml einer 1.6 N Lösung von Butyllithium in Hexan zugetropft und 5 Minuten bei gleichbleibender Temperatur gerührt. Die Reaktionslösung wurde mit 3.23 ml Cyclobutanon versetzt und unter Rühren auf Raumtemperatur kommen gelassen. Nach der Zugabe von 1.24 ml Essigsäure wurden die flüchtigen Bestandteile unter Vakuum entfernt. Der Rückstand wurde in einer Mischung aus 50 ml Ethylacetat und 50 ml wässriger Natriumhydrogencarbonat-Lösung aufgenommen und die wässrige Phase erneut mit 50 ml Ethylacetat extrahiert. Die vereinten organischen Phasen wurden über MgSO₄ getrocknet und einrotiert. Das so erhaltene Rohprodukt wurde aus Ethylacetet/n-Heptan umkristallisiert, anschließend in 20 ml Methanol gelöst und unter Zusatz von 0.36 g Pd(OH)₂/C 20 % bei 60 °C für 3 Stunden unter Wasserstoff-Atmosphäre gerührt. Nach Filtration von den festen Rückständen wurde das Filtrat unter vermindertem Druck vom Lösungsmittel befreit. Man erhielt so das Produkt (1.07 g) mit dem Molekulargewicht 165.2 g / mol (C₅H₁₁NO₃S).

### Cyclohexyl-(8,8-dioxo-5-oxa-8lambda*6*-thia-7-aza-spiro[3.5]non-6-en-6-yl)-amin

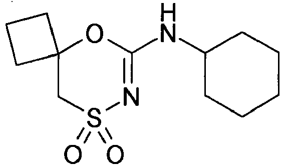

Unter Inertgas wurden 200 mg (1-Hydroxy-cyclobutyl)-methansulfonamid in 2.5 ml NMP gelöst, bei -10 °C mit 0.61 ml einer 2 N Lösung von Natrium-bis(trimethylsilyl)-amid in THF und nach 5 Minuten Rühren mit einer Lösung aus 0.19 ml Cyclohexylisothiocyanat in 0.5 ml NMP versetzt. Nach 40 Minuten Rühren bei gleichbleibender Temperatur wurden 215 mg N-Bromsuccinimid in 4 Portionen innerhalb von 5 Minuten zugegeben und die Reaktionslösung weitere 15 Minuten gerührt. Das Reaktionsgemisch wurde mit 60 ml Wasser verdünnt und 2 x mit 20 ml Ethylacetat extrahiert. Die vereinten organischen Phasen wurden mit gesättigter wässriger Natriumchlorid-Lösung gewaschen, über MgSO₄ getrocknet und einrotiert. Der Rückstand wurde im Reinigungslabor über präparative HPLC gereinigt. Nach Lyophilisation der produkthaltigen Fraktionen erhielt man so das Produkt (114.7 mg) mit dem Molekulargewicht 272.4 g / mol (C₁₂H₂₀N₂O₃S); MS (ESI): m / e = 273 (M+H+).

Die Verbindungen 11 und 12 wurden mit dieser Herstellungsvorschrift synthetisiert:
Cyclohexyl-(9,9-dioxo-6-oxa-9lambda6-thia-8-aza-spiro[4.5]dec-7-en-7-yl)-amin
Cyclohexyl-(4,4-dioxo-1-oxa-4lambda6-thia-3-aza-spiro[5.5]undec-2-en-2-yl)-amin

### (S)-3-(8,8-Dioxo-5-oxa-8lambda6-thia-7-aza-spiro[3.5]non-6-en-6-ylamino)-3-phenyl-propan-1-ol

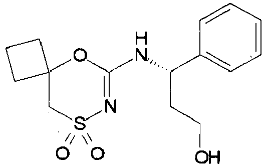

Die Darstellung erfolgte analog der oben beschriebenen Synthese der Verbindung 10. Die Einführung des Aminoalkohols wird nachfolgend beschrieben.

### (S)-3-(tert-Butyl-dimethyl-silanyloxy)-1-phenyl-propylamin

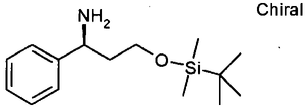

Es wurden 1.50 g (S)-3-Amino-3-phenylpropan-1-ol Hydrochlorid in 20 ml Dichlormethan vorgelegt und anschließend 2.77 ml Triethylamin und 1.30 g tert-Butyldimethylchlorsilan zugegeben. Nach 3 Stunden Rühren bei Raumtemperatur wurde die Reaktionslösung 3 x mit 30 ml Wasser extrahiert, über eine Phasenseparatorkartusche (Isolute®) getrocknet, einrotiert und im Hochvakuum getrocknet. Man erhielt so das Produkt (2.06 g) mit dem Molekulargewicht 265.5 g / mol (C₁₅H₂₇NOSi); MS (ESI): m / e = 266 (M+H+).

### (S)-3-(8,8-Dioxo-5-oxa-8lambda6-thia-7-aza-spiro[3.5]non-6-en-6-ylamino)-3-phenyl-propan-1-ol

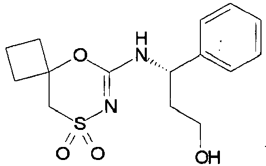

345 mg (S)-3-(tert-Butyl-dimethyl-silanyloxy)-1-phenyl-propylamin wurden in 3 ml Dichlormethan gelöst und mit 256 mg 1,1'-Thiocarbonyldiimidazol versetzt und 20 Minuten gerührt. Die Reaktionslösung wurde mit einem Gemisch aus 20 ml Diethylether und 20 ml n-Pentan versetzt und 3x mit 30 ml Wasser gewaschen, über MgSO₄ getrocknet, einrotiert und der Rückstand in 1 ml NMP gelöst.

Unter Inertgas wurden 192 mg (1-Hydroxy-cyclobutyl)-methansulfonamid in 1.5 ml NMP gelöst, bei -10 °C mit 0.58 ml einer 2 N Lösung von Natrium-bis(trimethylsilyl)-amid in THF und nach 5 Minuten Rühren mit der oben hergestelleten Isothiocyanat-Lösung versetzt. Nach 30 Minuten Rühren wurden 207 mg N-Bromsuccinimid in 4 Portionen zugesetzt und die resultierende Reaktionslösung 30 Minuten bei gleichbleibender Temperatur gerührt. Das Reaktionsgemisch wurde mit 50 ml Wasser verdünnt und 2 x mit 20 ml Ethylacetat extrahiert. Die vereinten organischen Phasen wurden mit 20 ml gesättigter wässriger Ammoniumchlorid-Lösung gewaschen, über MgSO₄ getrocknet und einrotiert. Der Rückstand wurde in 3 ml Methanol gelöst, mit 0.30 ml konzentrierter Salzsäure versetzt und für 1.5 Stunden gerührt. Die Reaktionslösung wurde mit 15 ml Wasser und Ethylacetat versetzt, die organische Phase über MgSO₄ getrocknet, einrotiert und der Rückstand im Reinigungslabor über präparative HPLC gereinigt. Die produkthaltigen Fraktionen wurden vereinigt, unter vermindertem Druck vom Lösungsmittel befreit und der wässrige Rückstand lyophilisiert. Zur Spaltung des teilweise entstandenen Trifluoressigsäureethylesters wurde das Lyophilisat nochmals in Methanol gelöst, mit 0.5 ml einer 4 N Lösung von Salzsäure in Dioxan versetzt und 6 Stunden bei Raumtemperatur gerührt. Die Lösung wurde mit 10 ml Acetonitril versetzt, einrotiert, der Rückstand in etwas Acetonitril gelöst, mit Wasser versetzt und lyophilisiert. So erhielt man das Produkt (187.4 mg) mit dem Molekulargewicht 324.4 g / mol (C₁₅H₂₀N₂O₄S); MS (ESI): m /e = 325 (M+H⁺).

Die Verbindungen 13, 15 und 16 wurden mit dieser Herstellungsvorschrift synthetisiert:
(S)-3-(6,6-Dimethyl-4,4-dioxo-5,6-dihydro-4H-4lambda6-[1,4,3]oxathiazin-2-ylamino)-3-phenyl-propan-1-ol
(S)-3-(9,9-Dioxo-6-oxa-9lambda6-thia-8-aza-spiro[4.5]dec-7-en-7-ylamino)-3-phenyl-propan-1-ol
(S)-3-(4,4-Dioxo-1-oxa-4lambda6-thia-3-aza-spiro[5.5]undec-2-en-2-ylamino)-3-phenyl-propan-1-ol

### Cyclohexyl-(5,5,6,6-tetramethyl-4,4-dioxo-5,6-dihydro-4H-4lambda6-[1,4,3]oxathiazin-2-yl)-amin

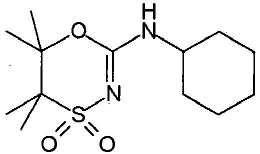

### 5,5,6,6-Tetramethyl-2-(4-nitro-phenoxy)-5,6-dihydro-1,4,3-oxathiazin-4,4-dioxid

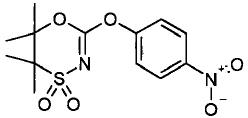

7.40 g Chlorsulfonylisocyanat und 7.27 g 4-Nitrophenol wurden in 75 ml Dichlormethan gelöst und für 90 Minuten bei Raumtemperatur gerührt. Die Reaktionslösung wurde unter vermindertem Druck vom Lösungsmittel befreit, der Rückstand in 50 ml THF gelöst und unter Inertgas und bei -78 °C mit 2.18 g Natriumhydrid versetzt. Nach 5 Minuten Rühren wurden 4.38 g 2,3-Dimethyl-2-buten zugegeben und die Reaktionsmischung, wegen beginnender Gasentwicklung langsam, auf 35 °C erhitzt. Nach 30 Minuten Rühren wurde die Suspension auf Eis gegeben und das resultierende THF/Wasser-Gemisch 2 x mit 100 ml Ethylacetat extrahiert. Die vereinten organischen Phasen wurden über Na₂SO₄ getrocknet und einrotiert. Der Rückstand wurde im Reinigungslabor über präparative HPLC gereinigt. Nach Lyophilisation der produkthaltigen Fraktionen erhielt man so das Produkt (2.76 g) mit dem Molekulargewicht 328.4 g / mol (C₁₃H₁₆N₂O₆S).

### Cyclohexyl-(5,5,6,6-tetramethyl-4,4-dioxo-5,6-dihydro-4H-4lambda6-[1,4,3]oxathiazin-2-yl)-amin

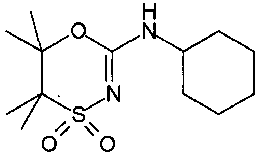

200 mg 5,5,6,6-Tetramethyl-2-(4-nitro-phenoxy)-5,6-dihydro-1,4,3-oxathiazin-4,4-dioxid und 60 mg Cyclohexylamin wurden in 2 ml Dichlormethan gelöst und 15 Minuten bei Raumtemperatur gerührt. Anschließend wurde die Reaktionslösung mit 50 ml Dichlormethan verdünnt, 2 x mit 50 ml 0.1 N wässriger Natriumhydroxid-Lösung, 2 x mit 50 ml 1 N wässriger Salzsäure und mit 50 ml gesättigter wässriger Natriumhydrogencarbonat-Lösung gewaschen, über Na₂SO₄ getrocknet und einrotiert. So erhielt man das Produkt (41 mg) mit dem Molekulargewicht 288.4 g / mol (C₁₃H₂₄N₂O₃S); MS (ESI): m / e = 289 (M+H⁺).

### Bicyclo[2.2.2]oct-1-yl-(5,5,6,6-tetramethyl-4,4-dioxo-5,6-dihydro-4H-4lambda6-[1,4,3]oxathiazin-2-yl)-amin

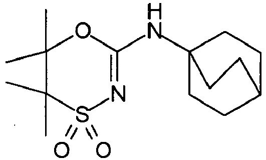

Das verwendete Amin wurde wie folgt hergestellt:

### 1-Amino-bicyclo[2.2.2]octan

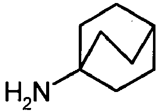

Helvetica Chimica Acta 1964, 47(2), 564-567 .

300 mg 5,5,6,6-Tetramethyl-2-(4-nitro-phenoxy)-5,6-dihydro-1,4,3-oxathiazin-4,4-dioxid und 163 mg 1-Amino-bicyclo[2.2.2]octan Hydrochlorid wurden in einer Mischung aus 5 ml Dichlormethan und 0.38 ml N,N-Diisopropylethylamin gelöst und 72 Stunden bei Raumtemperatur gerührt. Anschließend wurde die Reaktionslösung einrotiert und der Rückstand im Reinigungslabor über präparative HPLC gereinigt. Nach Lyophilisation der produkthaltigen Fraktionen erhielt man so das Produkt (166 mg) mit dem Molekulargewicht 314.5 g / mol (C₁₅H₂₆N₂O₃S); MS (ESI): m / e = 315 (M+H⁺).

### (2R,3aS,5S,6aS)-Hexahydro-2,5-methano-pentalen-3a-yl-(5,5,6,6-tetramethyl-4,4-dioxo-5,6-dihydro-4H-4lambda6-[1,4,3]oxathiazin-2-yl)-amin

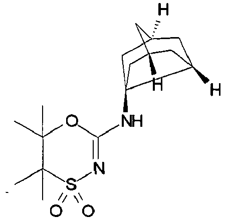

205 mg 5,5,6,6-Tetramethyl-2-(4-nitro-phenoxy)-5,6-dihydro-1,4,3-oxathiazin-4,4-dioxid und 120 mg 3-Aminonoradamantan Hydrochlorid wurden in einer Mischung aus 5 ml Dichlormethan und 0.32 ml N,N-Diisopropylethylamin gelöst und 16 Stunden bei Raumtemperatur gerührt. Anschließend wurde die Reaktionslösung mit 50 ml Dichlormethan verdünnt, 2 x mit 50 ml 0.1 N wässriger Natriumhydroxid-Lösung, 2 x mit 50 ml 1 N wässriger Salzsäure und mit 50 ml gesättigter wässriger Natriumhydrogencarbon-Lösung gewaschen und das Rohprodukt mit LCMS überprüft. Da die Umsetzung noch unvollständig war, wurde der Rückstand erneut in 5 ml Dichlormethan und 0.32 ml N,N-Diisopropylethylamin gelöst und für weitere 16 Stunden gerührt. Die Reaktionslösung wurde mehrmals mit 10 %iger wässriger Ammoniak-Lösung gewaschen, über Na₂SO₄ getrocknet und einrotiert. So erhielt man das Produkt (159 mg) mit dem Molekulargewicht 326.5 g/ mol (C₁₆H₂₆N₂O₃S); MS (ESI): m / e = 327 (M+H⁺).

### [(S)-1-(2-Fluor-phenyl)-ethyl]-(5,5,6,6-tetramethyl-4,4-dioxo-5,6-dihydro-4H-4lambda6-[1,4,3]oxathiazin-2-yl)-amin

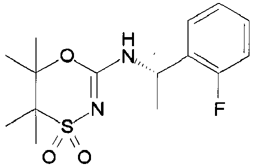

200 mg 5,5,6,6-Tetramethyl-2-(4-nitro-phenoxy)-5,6-dihydro-1,4,3-oxathiazin-4,4-dioxid und 111 mg (S)-1-(2-Fluoro-phenyl)-ethylamin wurden in 1 ml Dichlormethan und 0.10 ml N,N-Diisopropylethylamin gelöst und für 16 Stunden bei Raumtemperatur gerührt. Anschließend wurde die Reaktionslösung mit 50 ml Dichlormethan verdünnt, 3 x mit 30 ml 10 %iger wässriger Ammoniak-Lösung, 3 x mit 30 ml 1 N wässriger Salzsäure und mit 30 ml gesättigter wässriger Natriumhydrogencarbon-Lösung gewaschen, über Na₂SO₄ getrocknet und einrotiert. So erhielt man das Produkt (194 mg) mit dem Molekulargewicht 328.4 g / mol (C₁₅H₂₁FN₂O₃S); MS (ESI): m / e = 329 (M+H⁺).

### 3-[(S)-3-Hydroxy-1-(5,5,6,6-tetramethyl-4,4-dioxo-5,6-dihydro-4H-4lambda6-[1,4,3]oxathiazin-2-ylamino)-propyl]-benzonitril

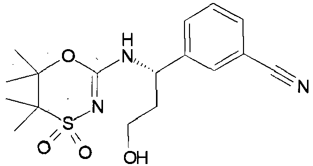

### (Sₛ)-2-Methyl-propan-2-sulfinsäure[3-(tert-butyl-dimethyl-silanyloxy)-prop-(E)-yliden]-amid

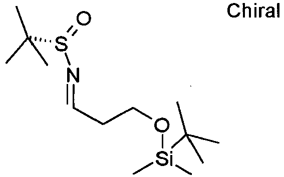

Unter Inertgas wurden 2.83 g (S)-(-)-2-Methyl-propan-2-sulfinsäureamid 60 ml Dichlormethan vorgelegt und unter Zusatz von 4.00 g 3-(tert-Butyl-dimethyl-silanyloxy)-propionaldehyd und 8.48 g wasserfreiem Kupfersulfat für 16 Stunden bei Raumtemperatur gerührt. Die Vollständigkeit der Umsetzung wurde mit LCMS geprüft und die Reaktion für weitere 24 Stunden gerührt. Nach Filtration von den festen Rückständen wurde das Filtrat unter vermindertem Druck vom Lösungsmittel befreit. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (5.03 g) mit dem Molekulargewicht 291.5 g / mol (C₁₃H₂₉NO₂SSi); MS (ESI): m / e = 292 (M+H⁺).

### (Sₛ)-2-Methyl-propan-2-sulfinsäure[(S)-3-(tert-butyl-dimethyl-silanyloxy)-1-(3-cyano-phenyl)-propyl]-amid

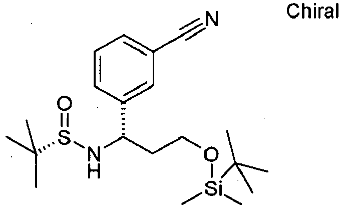

In einem unter Inertgas ausgeheizten Kolben wurden 9.73 ml einer 14 %igen Lösung des Isopropylmagnesiumchlorid/Lithiumchlorid-Komplexes in THF (d = 0.951 g / ml) vorgelegt und und auf-15 °C gekühlt. Nach der Zugabe einer Lösung von 1.56 g 3-Brombenzonitril in 5 ml THF wurde der Ansatz innerhalb von 30 Minuten auf eine Temperatur von -5 °C aufwärmen gelassen. In einem weiteren unter Inertgas ausgeheizten Kolben wurde 1.00 g (Sₛ)-2-Methyl-propan-2-sulfinsäure[3-(tert-butyl-dimethyl-silanyloxy)-prop-(E)-yliden]-amid in 50 ml Toluol gelöst und bei einer Temperatur von -78 °C die Lösung aus dem ersten Kolben tropfenweise zugegeben. Die Reaktionslösung wurde für 16 Stunden gerührt und währenddessen auf Raumtemperatur kommen gelassen. Anschließend wurde die Ansatzlösung mit 5 ml gesättigter wässriger Ammoniumchlorid-Lösung versetzt und unter vermindertem Druck vom Lösungsmittel befreit. Der Rückstand wurde in 50 ml Wasser und 50 ml Ethylacetat aufgenommen, die organische Phase über Na₂SO₄ getrocknet und einrotiert. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (0.25 g) mit dem Molekulargewicht 394.7 g / mol (C₂₀H₃₄N₂O₂SSi); MS (ESI): m / e = 395 (M+H⁺).

### 3-((S)-1-Amino-3-hydroxy-propyl)-benzonitril Hydrochlorid

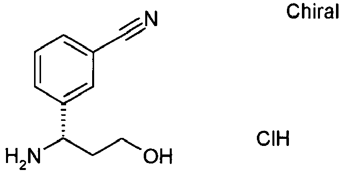

430 mg (Sₛ)-2-Methyl-propan-2-sulfinsäure[(S)-3-(tert-butyl-dimethyl-silanyloxy)-1-(3-cyano-phenyl)-propyl]-amid wurden in 5 ml Methanol gelöst und nach Zugabe von 1.36 ml einer 4 N Salzsäure in Dioxan 1 Stunde bei Raumtemperatur gerührt. Das Lösungsmittel wurde unter vermindertem Druck entfernt und das Rohprodukt ohne weitere Reinigung in die nächste Reaktion eingesetzt. Es wurde das Hydrochlorid des Produktes mit dem Molekulargewicht 176.2 g / mol (C₁₀H₁₂N₂O); MS (ESI): m / e = 177 (M+H⁺) erhalten.

### 3-[(S)-3-Hydroxy-1-(5,5,6,6-tetramethyl-4,4-dioxo-5,6-dihydro-4H-4lambda6-[1,4,3]oxathiazin-2-ylamino)-propyl]-benzonitril

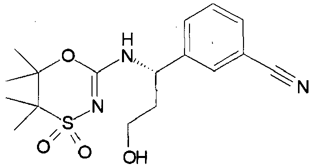

100 mg 5,5,6,6-Tetramethyl-2-(4-nitro-phenoxy)-5,6-dihydro-1,4,3-oxathiazin-4,4-dioxid und 97 mg 3-((S)-1-Amino-3-hydroxy-propyl)-benzonitril Hydrochlorid wurden in 5 ml Dichlormethan und 0.25 ml N,N-Diisopropylethylamin gelöst und für 16 Stunden bei Raumtemperatur gerührt. Anschließend wurde die Reaktionslösung mit 20 ml Dichlormethan verdünnt, mit 10 ml 10 %iger wässriger Ammoniak-Lösung und mit 10 ml 1 N wässriger Salzsäure gewaschen. Beide wässrige Phasen wurden nochmals mit 20 ml Ethylacetat extrahiert, die vereinten organischen Phasen über Na₂SO₄ getrocknet und einrotiert. Der Rückstand wurde im Reinigungslabor über präparative HPLC gereinigt. Nach Lyophilisation der produkthaltigen Fraktionen erhielt man so das Produkt (66 mg) mit dem Molekulargewicht 365.5 g / mol (C₁₇H₂₃N₃O₄S), MS (ESI): m / e = 366 (M+H+).

Die Synthese wurde in Analogie zur den in der Literatur beschriebenen chiralen Synthesen durchgeführt. Das Enantiomerenverhältnis des Produktes beträgt 95:5. Es wird davon ausgegangen, dass das S-Enantiomer in Überschuss entstanden ist.
Tetrahedron 1999, 8883
Journal of Organic Chemistry 2001, 8772
Tetrahedron Letters 2001, 2051

### (S)-4-(2-Chlor-phenyl)-2-methyl-4-(5,5,6,6-tetramethyl-4,4-dioxo-5,6-dihydro-4H-4lambda6-[1,4,3]oxathiazin-2-ylamino)-butan-2-ol

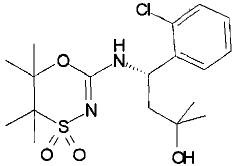

### (S)-3-(2-Chlor-phenyl)-3-(5,5,6,6-tetramethyl-4,4-dioxo-5,6-dihydro-4H-4lambda*6*-1,4,3-oxathiazin-2-ylamino)-propionsäuremethylester

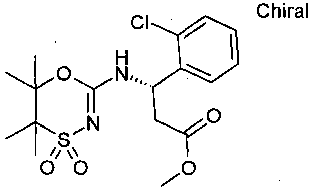

200 mg 5,5,6,6-Tetramethyl-2-(4-nitro-phenoxy)-5,6-dihydro-1,4,3-oxathiazin-4,4-dioxid und 130 mg (S)-3-Amino-3-(2-chlor-phenyl)-propionsäuremethylester wurden in 5 ml Dichlormethan und 0.52 ml N,N-Diisopropylethylamin gelöst und für 16 Stunden bei Raumtemperatur gerührt. Anschließend wurde die Reaktionslösung mit 20 ml Dichlormethan verdünnt und mehrmals mit 20 ml 25 %iger wässriger Ammoniak-Lösung gewaschen, bis die organische Phase farblos war. Die organische Phase wurde über Na₂SO₄ getrocknet und einrotiert. Man erhielt so das Produkt (191 mg) mit dem Molekulargewicht 402.9 g / mol (C₁₇H₂₃ClN₂O₅S); MS (ESI): m / e = 403 (M+H⁺).

### (S)-4-(2-Chlor-phenyl)-2-methyl-4-(5,5,6,6-tetramethyl-4,4-dioxo-5,6-dihydro-4H-4lambda6-[1,4,3]oxathiazin-2-ylamino)-butan-2-ol

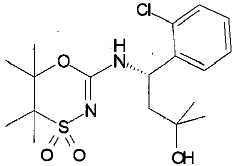

Unter Inertgas wurden 191 mg (S)-3-(2-Chlor-phenyl)-3-(5,5,6,6-tetramethyl-4,4-dioxo-5,6-dihydro-4H-4lambda*6*-1,4,3-oxathiazin-2-ylamino)-propionsäuremethylester in 4 ml THF gelöst und mit 0.66 ml einer 3 N Lösung von Methylmagnesiumbromid in Diethylether versetzt. Nach 30 Minuten Rühren bei Raumtemperatur wurde die Reaktionslösung einrotiert und der Rückstand im Reinigungslabor über präparative HPLC gereinigt. Nach Lyophilisation der produkthaltigen Fraktionen erhielt man so das Produkt (35 mg) mit dem Molekulargewicht 402.9 g / mol (C₁₈H₂₇ClN₂O₄S); MS (ESI): m / e = 403 (M+H⁺).

### [(S)-1-(2-Chlor-phenyl)-ethyl]-(5,5,6,6-tetramethyl-4,4-dioxo-5,6-dihydro-4H-4lambda6-[1,4,3]oxathiazin-2-yl)-amin

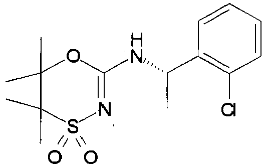

Unter Inertgas wurden 200 mg 5,5,6,6-Tetramethyl-2-(4-nitro-phenoxy)-5,6-dihydro-1,4,3-oxathiazin-4,4-dioxid und 176 mg (S)-1-(2-Chlor-phenyl)-ethylamin Hydrochlorid wurden in 1 ml Dichlormethan und 0.21 ml N,N-Diisopropylethylamin gelöst und für 16 Stunden bei Raumtemperatur gerührt. Anschließend wurde die Reaktionslösung mit 50 ml Dichlormethan verdünnt, 3 x mit 30 ml 10 %iger wässriger Ammoniak-Lösung, 3 x mit 30 ml 1 N wässriger Salzsäure und mit 30 ml gesättigter wässriger Natriumhydrogencarbon-Lösung gewaschen, über Na₂SO₄ getrocknet und einrotiert. So erhielt man das Produkt (171 mg) mit dem Molekulargewicht 344.94 g / mol (C₁₅H₂₁ClN₂O₃S); MS (ESI): m / e = 345 (M+H⁺).

### (1R,3R)-3-(5,5,6,6-Tetramethyl-4,4-dioxo-5,6-dihydro-4H-4lambda6-[1,4,3]oxathiazin-2-ylamino)-cyclohexanol

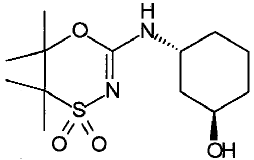

Das verwendete Amin wurde wie folgt hergestellt:

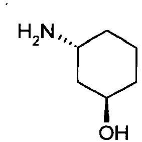

### (1R,3R)-3-Amino-cyclohexanol

P. Bernardelli, M. Bladon, E. Lorthiois, A.C. Manage, F. Vernige, R. Wriggleswort, Tetrahedron: Asymmetry 15 2004, 1451-1455
L.M. Levy, G. de Gonzalo, V. Gotor, Tetrahedron: Asymmetry 15 2004, 2051-2056

Unter Eiskühlung wurden 200 mg 5,5,6,6-Tetramethyl-2-(4-nitro-phenoxy)-5,6-dihydro-1,4,3-oxathiazin-4,4-dioxid und 88 mg (1R,3R)-3-Amino-cyclohexanol in 3 ml Dichlormethan und 0.26 ml N,N-Diisopropylethylamin gelöst und für 1.5 Stunden bei Raumtemperatur gerührt. Anschließend wurde die Reaktionslösung einrotiert und der Rückstand im Reinigungslabor über präparative HPLC gereinigt. Nach Lyophilisation der produkthaltigen Fraktionen erhielt man so das Produkt (136 mg) mit dem Molekulargewicht 304.4 g / mol (C₁₃H₂₄N₂O₄S); MS (ESI): m / e = 305 (M+H⁺).

### (4-Fluor-bicyclo[2.2.2]oct-1-yl)-(5,5,6,6-tetramethyl-4,4-dioxo-5,6-dihydro-4H-4lambda6-[1,4,3]oxathiazin-2-yl)-amin

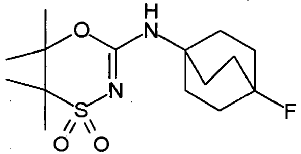

Die Synthese des Eduktes 2-(2,6-Difluoro-phenoxy)-5,5,6,6-tetramethyl-5,6-dihydro-1,4,3-oxathiazin 4,4-dioxid erfolgte analog zur bereits beschriebenen Herstellung von 5,5,6,6-Tetramethyl-2-(4-nitro-phenoxy)-5,6-dihydro-1,4,3-oxathiazin-4,4-dioxid.

Das verwendete Amin wurde wie folgt hergestellt:

### Amino-4-fluor-bicyclo[2.2.2]octan

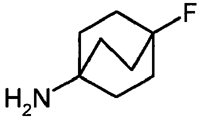

Journal of Organic Chemistry 1982, 47(15), 2951-2957 .

120 mg 2-(2,6-Difluoro-phenoxy)-5,5,6,6-tetramethyl-5,6-dihydro-1,4,3-oxathiazin 4,4-dioxid und 67 mg Amino-4-fluor-bicyclo[2.2.2]octan wurden in 2 ml Dichlormethan und 0.13 ml N,N-Diisopropylethylamin gelöst und 15 Minuten bei Raumtemperatur gerührt. Anschließend wurde die Reaktionslösung mit 50 ml Dichlormethan verdünnt, 2 x mit 50 ml 0.1 N wässriger Natriumhydroxid-Lösung, 2 x mit 50 ml 1 N wässriger Salzsäure und mit 50 ml gesättigter wässriger Natriumhydrogencarbon-Lösung gewaschen, über Na₂SO₄ getrocknet und einrotiert. Der Rückstand wurde im Reinigungslabor über präparative HPLC gereinigt. Nach Lyophilisation der produkthaltigen Fraktionen erhielt man so das Produkt (12.1 mg) mit dem Molekulargewicht 332.4 g / mol (C₁₅H₂₅FN₂O₃S); MS (ESI): m / e = 333 (M+H⁺).

Die Verbindungen 24 - 26 wurden mit dieser Herstellungsvorschrift synthetisiert:
(S)-3-Phenyl-3-(5,5,6,6-tetramethyl-4,4-dioxo-5,6-dihydro-4H-4lambda6-[1,4,3]oxathiazin-2-ylamino)-propan-1-ol
(S)-3-(2-Chlor-phenyl)-3-(5,5,6,6-tetramethyl-4,4-dioxo-5,6-dihydro-4H-4lambda6-[1,4,3]oxathiazin-2-ylamino)-propan-1-ol
(S)-3-(4-Fluor-phenyl)-3-(5,5,6,6-tetramethyl-4,4-dioxo-5,6-dihydro-4H-4lambda6-[1,4,3]oxathiazin-2-ylamino)-propan-1-ol

### (S)-2-Methyl-4-pyridin-3-yl-4-(5,5,6,6-tetramethyl-4,4-dioxo-5,6-dihydro-4H-4lambda6-[1,4,3]oxathiazin-2-ylamino)-butan-2-ol

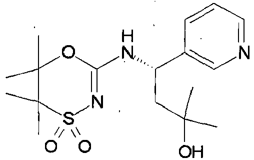

### (S)-3-Pyridin-3-yl-3-(5,5,6,6-tetramethyl-4,4-dioxo-5,6-dihydro-4H-4lambda*6*-1,4,3-oxathiazin-2-ylamino)-propionsäuremethylester Trifluoracetat

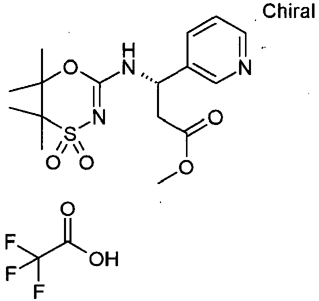

195 mg 2-(2,6-Difluoro-phenoxy)-5,5,6,6-tetramethyl-5,6-dihydro-1,4,3-oxathiazin 4,4-dioxid und 132 mg (S)-3-Amino-3-pyridin-3-yl-propionsäuremethylester wurden in 2 ml Dichlormethan und 0.26 ml N,N-Diisopropylethylamin gelöst und für 48 Stunden bei Raumtemperatur gerührt. Nach dem Einrotieren wurde der Rückstand im Reinigungslabor über präparative HPLC gereinigt. Nach Lyophilisation der produkthaltigen Fraktionen erhielt man so das Trifluoracetat des Produktes (97.4 mg) mit dem Molekulargewicht 369.4 g / mol (C₁₆H₂₃N₃O₅S); MS (ESI): m / e = 370 (M+H⁺).

### (S)-2-Methyl-4-pyridin-3-yl-4-(5,5,6,6-tetramethyl-4,4-dioxo-5,6-dihydro-4H-4lambda6-[1,4,3]oxathiazin-2-ylamino)-butan-2-ol

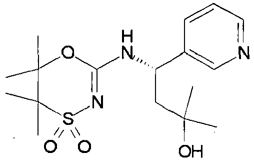

Unter Inertgas wurden 50 mg (S)-3-Pyridin-3-yl-3-(5,5,6,6-tetramethyl-4,4-dioxo-5,6-dihydro-4H-4lambda*6*-1,4,3-oxathiazin-2-ylamino)-propionsäuremethylester Trifluoracetat in 2 ml THF gelöst und mit 0.14 ml einer 3 N Lösung von Methylmagnesiumbromid in Diethylether versetzt. Nach 30 Minuten Rühren bei Raumtemperatur wurde die Reaktionslösung mit 10 ml Wasser und 30 ml Ethylacetat versetzt, die organische Phase mit 10 ml gesättigter Natriumchlorid-Lösung gewaschen, über Na₂SO₄ getrocknet und einrotiert. Der Rückstand wurde im Reinigungslabor über präparative HPLC gereinigt und die produkthaltigen Fraktionen unter vermindertem Druck vom Lösungsmittel befreit. So erhielt man das Produkt (25.1 mg) mit dem Molekulargewicht 369.5 g / mol (C₁₇H₂₇N₃O₄S); MS (ESI): m / e = 370 (M+H⁺).

### (1S,3R,5R,7S)-3-(5,5,6,6-Tetramethyl-4,4-dioxo-5,6-dihydro-4H-4lambda6-[1,4,3]oxathiazin-2-ylamino)-adamantan-1-ol

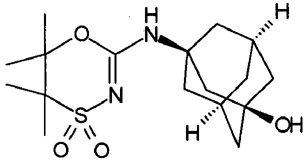

200 mg 2-(2,6-Difluoro-phenoxy)-5,5,6,6-tetramethyl-5,6-dihydro-1,4,3-oxathiazin 4,4-dioxid und 105 mg 3-Amino-1-adamantanol wurden in 2 ml Dichlormethan und 0.32 ml N,N-Diisopropylethylamin gelöst und für 16 Stunden bei Raumtemperatur gerührt. Nach dem Einrotieren wurde der Rückstand im Reinigungslabor über präparative HPLC gereinigt. Nach Lyophilisation der produkthaltigen Fraktionen erhielt man das Produkt (56 mg) mit dem Molekulargewicht 356.5 g / mol (C₁₇H₂₈N₂O₄S); MS (ESI): m / e = 357 (M+H⁺).

Die Verbindung 32 wurde mit dieser Herstellungsvorschrift synthetisiert:
(1S,3S)-3-(5,5,6,6-Tetramethyl-4,4-dioxo-5,6-dihydro-4H-4lambda6-[1,4,3]oxathiazin-2-ylamino)-cyclohexanol
(1S,3S)-3-Amino-cyclohexanol wurde wie folgt hergestellt:
   P. Bernardelli, M. Bladon, E. Lorthiois, A.C. Manage, F. Vernige, R. Wriggleswort, Tetrahedron: Asymmetry 15 2004, 1451-1455
   L.M. Levy, G. de Gonzalo, V. Gotor, Tetrahedron: Asymmetry 15 2004, 2051-2056

### ((S)-1-Phenyl-ethyl)-(5,5,6,6-tetramethyl-4,4-dioxo-5,6-dihydro-4H-4lambda6-[1,4,3]oxathiazin-2-yl)-amin

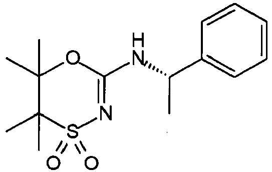

Eine Mischung aus 194 mg 2-(2,6-Difluoro-phenoxy)-5,5,6,6-tetramethyl-5,6-dihydro-1,4,3-oxathiazin 4,4-dioxid und 737 mg (S)-1-Phenyl-ethylamin wurde für 16 Stunden bei Raumtemperatur gerührt und anschließend im Reinigungslabor über präparative HPLC gereinigt. Die vereinten produkthaltigen Fraktionen wurden mit 25 % wässriger Ammoniak-Lösung alkalisch gestellt, 3 x mit 30 ml Ethylacetat extrahiert, die vereinten organischen Phasen über MgSO₄ getrocknet und einrotiert. Man erhielt so das Produkt (96 mg) mit dem Molekulargewicht 310.4 g / mol (C₁₅H₂₂N₂O₃S); MS (ESI): m / e = 311 (M+H⁺).

### 2-(4-Fluor-phenyl)-2-(5,5,6,6-tetramethyl-4,4-dioxo-5,6-dihydro-4H-4lambda6-[1,4,3]oxathiazin-2-ylamino)-ethansulfonsäureamid

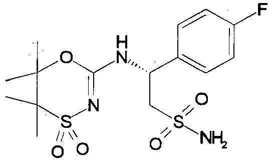

### N-tert-Butyl-methansulfonamid

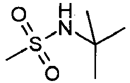

Zu einer Lösung von 7.74 ml Methansulfonylchlorid in 100 ml Dichlormethan wurden bei einer Temperatur von 0 °C tropfenweise 23.12 ml tert-Butylamin zugegeben. Nach vollständiger Zugabe wurde der Niederschlag abfiltriert und das Filtrat mit 100 ml 1 N wässriger Salzsäure gewaschen, die organische Phase über MgSO₄ getrocknet und das Lösungsmittel unter vermindertem Druck entfernt. Man erhielt das Produkt (14.70 g) mit dem Molekulargewicht 151.2 g / mol (C₅H₁₃NO₂S).

### 2-(4-Fluor-phenyl)-2-oxo-ethansulfonsäure-tert-butylamid

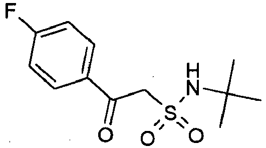

Unter Inertgas wurde 1.00 g N-tert-Butyl-methansulfonamid in 20 ml THF vorgelegt und anschließend bei einer Temperatur von -78 °C 9.00 ml einer 1.6 N Butyllithium-Lösung in Hexan tropfenweise zugegeben. Nach 5 Minuten Rühren unter Eiskühlung wurde erneut auf -78 °C abgekühlt und eine ebenfalls auf -78 °C gekühlte Lösung von 1.33 ml 4-Fluorbenzoesäuremethylester in 10 ml THF tropfenweise zugegeben. Die Mischung wurde auf Raumtemperatur kommen gelassen und 1 Stunde gerührt. Die Reaktionslösung wurde mit 0.82 ml Essigsäure versetzt, mit 50 ml Ethylacetat verdünnt und mit 40 ml gesättigter wässriger Natriumhydrogencarbonat-Lösung und 40 ml gesättigter wässriger Natriumchlorid-Lösung gewaschen. Die organische Phase wurde über MgSO₄ getrocknet und einrotiert. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (662 mg) mit dem Molekulargewicht 273.3 g / mol (C₁₂H₁₆FNO₃S).

### 2-Amino-2-(4-fluor-phenyl)-ethansulfonsäure-tert-butylamid

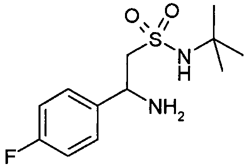

Eine Lösung aus 0.66 g 2-(4-Fluor-phenyl)-2-oxo-ethansulfonsäure-tert-butylamid, 1.85 g Ammoniumacetat und 0.17 g Natriumcyanoborhydrid in 12 ml Methanol wurde 17 Stunden bei 60 °C gerührt. Nach dem Entfernen des Lösungsmittels unter vermindertem Druck wurde der Rückstand in 50 ml Ethylacetat aufgenommen und 2 x mit 30 ml 0.5 N wässriger Natriumhydroxid-Lösung und 30 ml gesättigter wässriger Natriumchlorid-Lösung gewaschen. Die organische Phase wurde über MgSO₄ getrocknet und einrotiert.

Das Rohprodukt wurde einer chiralen Trennung unterzogen.
Säule: Chiralpak AD-H/39, 5 µm, 250 x 4.6 mm
Laufmittel: Ethanol : Methanol 1 : 1 + 0.1 % Diethylamin (30 °C, Fluss 1 ml / min)
Retentionszeiten: 4.787 min (Enantiomer 1), 10.635 min (Enantiomer 2)

Die produkthaltigen Fraktionen wurden jeweils vereinigt und einrotiert. Man erhielt so die beiden Produkte (Enantiomer 1: 269 mg/ Enantiomer 2: 241 mg) mit dem Molekulargewicht 274.4 g / mol (C₁₂H₁₉FN₂O₂S); MS (ESI): m / e = 275 (M+H⁺). Die absolute Konfiguration der beiden Produkte wurde nicht bestimmt.

### 2-(4-Fluor-phenyl)-2-(5,5,6,6-tetramethyl-4,4-dioxo-5,6-dihydro-4H-4lambda6-[1,4,3]oxathiazin-2-ylamino)-ethansulfonsäureamid

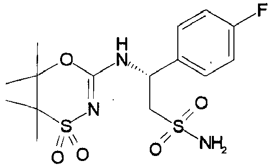

50 mg 2-Amino-2-(4-fluor-phenyl)-ethansulfonsäure-tert-butylamid Enantiomer 1 und 58 mg 2-(2,6-Difluoro-phenoxy)-5,5,6,6-tetramethyl-5,6-dihydro-1,4,3-oxathiazin 4,4-dioxid wurden in 1 ml Dichlormethan gelöst und für 5 Stunden bei Raumtemperatur gerührt. Die Vollständigkeit der Umsetzung wurde mit LCMS geprüft. Da noch keine Umsetzung stattgefunden hatte, wurden 60 µl N,N-Diisopropylethylamin zugegeben und über Nacht bei Raumtemperatur gerührt. Die Reaktionslösung wurde mit 20 ml Ethylacetat verdünnt und mit 10 ml 0.1 N Salzsäure und 10 ml gesättigter wässriger Natriumchlorid-Lösung gewaschen. Die organische Phase wurde über MgSO₄ getrocknet und einrotiert. Der Rückstand wurde in 1 ml Trifluoressigsäure gelöst und für 20 Stunden bei Raumtemperatur gelöst. Nach dem Entfernen des Lösungsmittels unter vermindertem Druck wurde das Rohprodukt im Reinigungslabor über präparative HPLC gereinigt. Nach Lyophilisation der produkthaltigen Fraktionen erhielt man das Produkt (25.8 mg) mit dem Molekulargewicht 407.5 g / mol (C₁₅H₂₂FN₃O₅S₂), MS (ESI): m / e = 408 (M+H⁺).

## Patentansprüche

1. Verbindungen der Formel I, 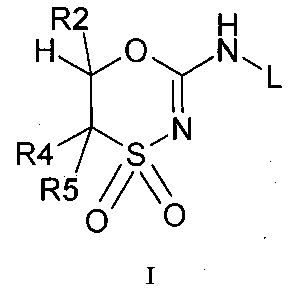 worin bedeuten
L R1, -CH(R10)(R11);
R10, R11 unabhängig voneinander H, F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₁-C₆)-Alkylen-(R6), (C₃-C₈)-Cycloalkylen-(R6), (C₁-C₆)-Alkylen-(C₃-C₈)-Cycloalkylen-(R6), (C₆-C₁₀)-Aryl, (C₁-C₆)-Alkylen-(C₆-C₁₀)-Aryl, -(C₆-C₁₀)-Heteroaryl, (C₁-C₆)-Alkylen-(C₆-C₁₀)-Heteroaryl;
wobei der Arylrest oder Heteroarylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)- Alkyl)₂, SF₅;
R6 OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R1 (C₆-C₁₀)-Aryl, (C₃-C₈)-Cycloalkyl, (C₃-C₈)-Carbocyclyl,
wobei der Arylrest, Cycloalkylrest oder Carbocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R2, R4, R5 unabhängig voneinander (C₁-C₆)-Alkyl, CF₃, CHF₂, CH₂F, (C₁-C₆)-Alkylen-OH, (C₁-C₆)-Alkylen-O-(C₁-C₆)-Alkyl, (C₆-C₁₀)-Aryl, (C₁-C₆)-Alkylen-O-(C₆-C₁₀)-Aryl" oder R4 und R5 bilden gemeinsam mit dem Kohlenstoffatom an das sie gebunden sind einen 3-8 gliedrigen, gesättigten carbocyclischen oder heterocyclischen Ring, der bis zu 2 weitere Heteroatome aus der Gruppe N, O oder S enthalten kann;
sowie deren pharmazeutisch verträgliche Salze.

2. Verbindungen der Formel I, gemäß Anspruch 1, **dadurch gekennzeichnet, dass** darin bedeuten
L R1;
R1 (C₆-C₁₀)-Aryl, (C₃-C₈)-Cycloalkyl, (C₃-C₈)-Carbocyclyl,
wobei der Arylrest, Cycloalkylrest oder Carbocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R2, R4, R5 unabhängig voneinander (C₁-C₆)-Alkyl, CF₃, CHF₂, CH₂F, (C₁-C₆)-Alkylen-OH, (C₁-C₆)-Alkylen-O-(C₁-C₆)-Alkyl, (C₆-C₁₀)-Aryl, (C₁-C₆)-Alkylen-O-(C₆-C₁₀)-Aryl" oder R4 und R5 bilden gemeinsam mit dem Kohlenstoffatom an das sie gebunden sind einen 3-8 gliedrigen, gesättigten carbocyclischen oder heterocyclischen Ring, der bis zu 2 weitere Heteroatome aus der Gruppe N, O oder S enthalten kann;
sowie deren pharmazeutisch verträgliche Salze.

3. Verbindungen der Formel I, gemäß Anspruch 1 oder 2, **dadurch gekennzeichnet, dass** darin bedeuten
L R1;
R1 (C₆-C₁₀)-Aryl, (C₃-C₈)-Cycloalkyl,
wobei der Arylrest oder Cycloalkylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R2, R4, R5 unabhängig voneinander (C₁-C₆)-Alkyl, CF₃, CHF₂, CH₂F, (C₁-C₆)-Alkylen-OH, (C₁-C₆)-Alkylen-O-(C₁-C₆)-Alkyl, (C₆-C₁₀)-Aryl, (C₁-C₆)-Alkylen-O-(C₆-C₁₀)-Aryl, oder R4 und R5 bilden gemeinsam mit dem Kohlenstoffatom an das sie gebunden sind einen 3-8 gliedrigen, gesättigten carbocyclischen Ring;
sowie deren pharmazeutisch verträgliche Salze.

4. Verbindungen der Formel I, gemäß einem oder mehreren der Ansprüche 1 bis 3, **dadurch gekennzeichnet, dass** darin bedeuten
L R1;
R1 (C₃-C₈)-Cycloalkyl,
wobei der Cycloalkylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R2 (C₁-C₆)-Alkyl, Phenyl;
R4, R5 unabhängig voneinander (C₁-C₆)-Alkyl, oder R4 und R5 bilden gemeinsam mit dem Kohlenstoffatom an das sie gebunden sind einen 3-8 gliedrigen, gesättigten carbocyclischen Ring;
sowie deren pharmazeutisch verträgliche Salze.

5. Verbindungen der Formel I, gemäß einem oder mehreren der Ansprüche 1 bis 4, zur Anwendung als Arzneimittel.

6. Arzneimittel enthaltend Verbindungen der Formel I, gemäß einem oder mehreren der Ansprüche 1 bis 4.

7. Arzneimittel enthaltend Verbindungen der Formel I, gemäß einem oder mehreren der Ansprüche 1 bis 4 und mindestens einen weiteren Wirkstoff.

8. Arzneimittel, gemäß Anspruch 7, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Modulatoren des GPR40, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten,, CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten, Lipase/Amylase-Inhibitoren, PPAR-Modulatoren, RXR-Modulatoren oder TR-β-Agonisten oder Amphetamine enthält.

9. Verfahren zur Herstellung eines Arzneimittels enthaltend die Verbindungen der Formel I, gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

10. Verwendung der Verbindungen der Formel I, gemäß einem oder mehreren der Ansprüche 1 bis 4 zur Herstellung eines Medikaments zur Behandlung von Hyperglykämie.

11. Verwendung der Verbindungen der Formel I, gemäß einem oder mehreren der Ansprüche 1 bis 4 zur Herstellung eines Medikaments zur Behandlung des Diabetes.

12. Verwendung der Verbindungen der Formel I, gemäß einem oder mehreren der Ansprüche 1 bis 4 zur Herstellung eines Medikaments zur Behandlung von Insulin Resistenz.

13. Set (Kit) bestehend aus getrennten Packungen von
a) einer wirksamen Menge einer Verbindung der Formel I, gemäß einem oder mehreren der Ansprüche 1 bis 4 und
b) einer wirksamen Menge eines weiteren Arzneimittelswirkstoffs.

## Claims

1. Compounds of the formula I 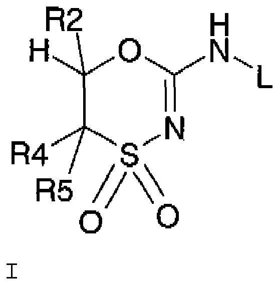 in which
L is R1, -CH(R10)(R11);
R10, R11 are each independently H, F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, COOH, COO-(C₁-C₆)-alkyl, CONH₂, CONH(C₁-C₆)-alkyl, CON((C₁-C₆)-alkyl)₂, SF₅, (C₁-C₆)-alkylene-(R6), (C₃-C₈)-cycloalkylene-(R6), (C₁-C₆)-alkylene-(C₃-C₈)-cycloalkylene-(R6), (C₆-C₁₀)-aryl, (C₁-C₆)-alkylene-(C₆-C₁₀)-aryl, -(C₆-C₁₀)-heteroaryl, (C₁-C₆)-alkylene-(C₆-C₁₀)-heteroaryl;
where the aryl radical or heteroaryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆-alkyl, CONH₂, CONH(C₁-C₆)-alkyl, CON((C₁-C₆)-alkyl)₂, SF₅;
R6 is OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON ((C₁-C₆)-alkyl)₂, SF₅;
R1 is (C₆-C₁₀)-aryl, (C₃-C₈)-cycloalkyl, (C₃-C₈)-carbocyclyl,
where the aryl radical, cycloalkyl radical or carbocyclyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R2, R4, R5 are each independently (C₁-C₆)-alkyl, CF₃, CHF₂, CH₂F, (C₁-C₆)-alkylene-OH, (C₁-C₆)-alkylene-O-(C₁-C₆)-alkyl, (C₆-C₁₀)-aryl, (C₁-C₆)-alkylene-O-(C₆-C₁₀)-aryl, or R4 and R5 together with the carbon atom to which they are bonded form a 3-8-membered carbocyclic or heterocyclic ring which may contain up to 2 further heteroatoms from the group of N, 0 and S;
and pharmaceutically compatible salts thereof.

2. Compounds of the formula I according to Claim 1, **characterized in that**
L is R1;
R1 is (C₆-C₁₀)-aryl, (C₃-C₈) -cycloalkyl, (C₃-C₈)-carbocyclyl,
where the aryl radical, cycloalkyl radical or carbocyclyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R2, R4, R5 are each independently (C₁-C₆)-alkyl, CF₃, CHF₂, CH₂F, (C₁-C₆)-alkylene-OH, (C₁-C₆)-alkylene-O-(C₁-C₆)-alkyl, (C₆-C₁₀)-aryl, (C₁-C₆)-alkylene-O-(C₆-C₁₀)-aryl, or R4 and R5 together with the carbon atom to which they are bonded form a 3-8-membered saturated carbocyclic or heterocyclic ring which may contain up to 2 further heteroatoms from the group of N, O and S;
and pharmaceutically compatible salts thereof.

3. Compounds of the formula I according to Claim 1 or 2, **characterized in that**
L is R1;
R1 is (C₆-C₁₀)-aryl, (C₃-C₈)-cycloalkyl,
where the aryl radical or cycloalkyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₃, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R2, R4, R5 are each independently (C₁-C₆)-alkyl, CF₃, CHF₂, CH₂F, (C₁-C₆)-alkylene-OH, (C₁-C₆)-alkylene-O-(C₁-C₆)-alkyl, (C₆-C₁₀)-aryl, (C₁-C₆)-alkylene-O-(C₆-C₁₀)-aryl, or R4 and R5 together with the carbon atom to which they are bonded form a 3-8-membered saturated carbocyclic ring;
and pharmaceutically compatible salts thereof.

4. Compounds of the formula I according to one or more of Claims 1 to 3, **characterized in that**
L is R1;
R1 is (C₃-C₈)-cycloalkyl,
where the cycloalkyl radical may be mono-to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R2 is (C₁-C₆)-alkyl, phenyl;
R4, R5 are each independently (C₁-C₆)-alkyl, or R4 and R5 together with the carbon atom to which they are bonded form a 3-8-membered saturated carbocyclic ring;
and pharmaceutically compatible salts thereof.

5. Compounds of the formula I according to one or more of Claims 1 to 4 for use as medicaments.

6. Medicament comprising compounds of the formula I according to one or more of Claims 1 to 4.

7. Medicament comprising compounds of the formula I according to one or more of Claims 1 to 4 and at least one further active ingredient.

8. Medicament according to Claim 7, **characterized in that** it comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose 1,6-biphosphatase, modulators of glucose transporter 4, inhibitors of glutamine:fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, GPR40 modulators, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, decoupling protein 2 or 3 modulators, leptin agonists, DA agonists, lipase/amylase inhibitors, PPAR modulators, RXR modulators or TR β agonists or amphetamines.

9. Process for producing a medicament comprising the compounds of the formula I according to one or more of Claims 1 to 4, **characterized in that** the active ingredient is mixed with a pharmaceutically suitable carrier and this mixture is converted to a form suitable for administration.

10. Use of the compounds of the formula I according to one or more of Claims 1 to 4 for producing a medicament for treatment of hyperglycemia.

11. Use of the compounds of the formula I according to one or more of Claims 1 to 4 for producing a medicament for treatment of diabetes.

12. Use of the compounds of the formula I according to one or more of Claims 1 to 4 for producing a medicament for treatment of insulin resistance.

13. Set (kit) consisting of separate packages of
a) an effective amount of a compound of the formula I according to one or more of Claims 1 to 4 and
b) an effective amount of a further active medicament ingredient.

## Revendications

1. Composés de formule I, 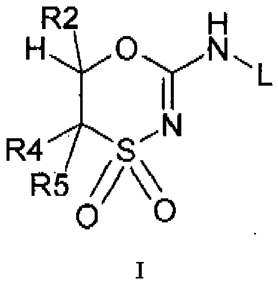 dans laquelle
L signifie R1, -CH(R10)(R11);
R10, R11 signifient, indépendamment l'un de l'autre H, F, Cl, Br, I, OH, CF₃, CHF₂, CH₂F, NO₂, CN,OCF₃, OCHF₂, O-(C₁-C₆)-alkyle, (C₁-C₆)-alkyle, NH₂, NH(C₁-C₆)-alkyle, N((C₁-C₆)-alkyle)₂, COOH, COO-(C₁-C₆) - alkyle, CONH₂, CONH(C₁-C₆)-alkyle, CON ((C₁-C₆)-alkyle)₂, SF₅, (C₁-C₆) -alkylène- (R6), (C₃-C₈)-cycloalkylène-(R6), (C₁-C₆)-alkylène-(C₃-C₈)-cycloalkylène-(R6), (C₆-C₁₀)-aryle, (C₁-C₆)-alkylène-(C₆-C₁₀)-aryle, - (C₆-C₁₀)-hétéroaryle, (C₁-C₆)-alkylène-(C₆-C₁₀)-hétéroaryle ;
le radical aryle ou hétéroaryle pouvant être monosubstitué à trisubstitué par F, Cl, Br, I, OH, CF₃, CHF₂, CH₂F, NO₂, CN, OCF₃, OCHF₂, O-(C₁-C₆)-alkyle, (C₁-C₆)-alkyle, NH₂, NH(C₁-C₆)-alkyle, N((C₁-C₆)-alkyle)₂, SO₂-CH₃, SO₂-NH₂, SO₂-NH(C₁-C₆)-alkyle, SO₂-N((C₁-C₆)-alkyle)₂, COOH, COO-(C₁-C₆)-alkyle, CONH₂, CONH(C₁-C₆)-alkyle, CON((C₁-C₆)-alkyle)₂, SF₅;
R6 signifie OH, CF₃, CHF₂, CH₂F, NO₂, CN, OCF₃, OCHF₂, O- (C₁-C₆)-alkyle, (C₁-C₆)-alkyle, NH₂, NH(C₁-C₆)-alkyle, N((C₁-C₆)-alkyle)₂, SO₂-CH₃, SO₂-NH₂, SO₂-NH(C₁-C₆)-alkyle, SO₂-N((C₁-C₆)-alkyle)₂, COOH, COO-(C₁-C₆)-alkyle, CONH₂, CONH(C₁-C₆)-alkyle, CON((C₁-C₆)-alkyle)₂, SF₅;
R1 signifie (C₆-C₁₀)-aryle, (C₃-C₈)-cycloalkyle, (C₃-C₈)-carbocyclyle,
le radical aryle, cycloalkyle ou carbocyclyle pouvant être monosubstitué à trisubstitué par F, Cl, Br, I, OH, CF₃, CHF₂, CH₂F, NO₂, CN, OCF₃, OCHF₂, O-(C₁-C₆)-alkyle, (C₁-C₆)-alkyle, NH₂, NH(C₁-C₆)-alkyle, N((C₁-C₆)-alkyle)₂, SO₂-CH₃, SO₂-NH₂, SO₂-NH(C₁-C₆)-alkyle, SO₂-N((C₁-C₆)-alkyle)₂, COOH, COO-(C₁-C₆)-alkyle, CONH₂, CONH(C₁-C₆)-alkyle, CON((C₁-C₆)-alkyle)₂, SF₅ ;
R2, R4, R5 signifient, indépendamment les uns des autres, (C₁-C₆)-alkyle, CF₃, CHF₂, CH₂F, (C₁-C₆)-alkylène-OH, (C₁-C₆)-alkylène-O-(C₁-C₆)-alkyle, (C₆-C₁₀) -aryle, (C₁-C₆)-alkylène-O- (C₆-C₁₀) -aryle, ou R4 et R5 forment, ensemble avec l'atome de carbone auquel ils sont liés, un cycle carbocyclique ou hétérocyclique saturé de 3-8 chaînons, qui peut contenir jusqu'à 2 autres hétéroatomes du groupe N, O ou S ;
ainsi que leurs sels pharmaceutiquement acceptables.

2. Composés de formule I, selon la revendication 1, **caractérisés en ce que**
L signifie R1 ;
R1 signifie (C₆-C₁₀) -aryle, (C₃-C₈)-cycloalkyle, (C₃-C₈)-carbocyclyle,
le radical aryle, cycloalkyle ou carbocyclyle pouvant être monosubstitué à trisubstitué par F, Cl, Br, I, OH, CF₃, CHF, CH₂F, NO₂, CN, OCF₃, OCHF₂, O-(C₁-C₆)-alkyle, (C₁-C₆)-alkyle, NH₂, NH(C₁-C₆)-alkyle, N((C₁-C₆)-alkyle)₂, SO₂-CH₃, SO₂-NH₂, SO₂-NH(C₁-C₆)-alkyle, SO₂-N((C₁-C₆)-alkyle)₂, COOH, COO-(C₁-C₆)-alkyle, CONH₂, CONH(C₁-C₆)-alkyle, CON((C₁-C₆)-alkyle)₂, SF₅;
R2, R4, R5 signifient, indépendamment les uns des autres, (C₁-C₅) -alkyle, CF₃, CHF₂, CH₂F, (C₁-C₆)-alkylène-OH, (C₁-C₆)-alkylène-O-(C₁-C₆)-alkyle, (C₆-C₁₀)-aryle, (C₁-C₆)-alkylène-O-(C₆-C₁₀)-aryle, ou R4 et R5 forment, ensemble avec l'atome de carbone auquel ils sont liés, un cycle carbocyclique ou hétérocyclique saturé de 3-8 chaînons, qui peut contenir jusqu'à 2 autres hétéroatomes du groupe N, O ou S ;
ainsi que leurs sels pharmaceutiquement acceptables ;

3. Composés de formule I, selon la revendication 1 ou 2, **caractérisés en ce que**
L signifie R1 ;
R1 signifie (C₆-C₁₀)-aryle, (C₃-C₈)-cycloalkyle,
le radical aryle ou cycloalkyle pouvant être monosubstitué à trisubstitué par F, Cl, Br, I, OH, CF₃, CHF₂, CH₂F, NO₂, CN, OCF₃, OCHF₂, 0- (C₁-C₆)-alkyle, (C₁-C₆)-alkyle, NH₂, NH(C₁-C₆)-alkyle, N((C₁-C₆)-alkyle)₂, SO₂-CH₃, SO₂-NH₂, SO₂-NH(C₁-C₆)-alkyle, SO₂*-*N((C₁-C₆)-alkyle)₂, COOH, COO-(C₁-C₆)-alkyle, CONH₂, CONH(C₁-C₆)-alkyle, CON(((C₁-C₆) -alkyle)₂, SF₅ ;
R2, R4, R5 signifient, indépendamment les uns des autres, (C₁-C₆)-alkyle, CF₃, CHF₂, CH₂F, (C₁-C₆)-alkylène-OH, (C₁-C₆)-alkylène-O- (C₁-C₆)-alkyle, (C₆-C₁₀)-aryle, (C₁-C₆) -alkylène-O-(C₆-C₁₀)-aryle, ou R4 et R5 forment, ensemble avec l'atome de carbone auquel ils sont liés, un cycle carbocyclique saturé de 3-8 chaînons ;
ainsi que leurs sels pharmaceutiquement acceptables.

4. Composés de formule I selon l'une ou plusieurs des revendications 1 à 3, **caractérisés en ce que**
L signifie R1 ;
R1 signifie (C₃-C₈)-cycloalkyle,
le radical cycloalkyle pouvant être monosubstitué à trisubstitué par F, Cl, Br, I, OH, CF₃, CHF₂, CH₂F, NO₂, CN, OCF₃, OCHF₂, O-(C₁-C₆)-alkyle, (C₁-C₆)-alkyle, NH₂, NH(C₁-C₆)-alkyle, N((C₁-C₆)-alkyle)₂, SO₂-CH₃, SO₂-NH₂, SO₂-NH(C₁-C₆)-alkyle, SO₂-N((C₁-C₆)-alkyle)₂, COOH, COO-(C₁-C₆)-alkyle, CONH₂, CONH(C₁-C₆)-alkyle, CON((C₁-C₆)-alkyle)₂, SF₅ ;
R2 signifie (C₁-C₆)-alkyle, phényle ;
R4, R5 signifient, indépendamment l'un de l'autre, (C1-C6)-alkyle, ou R4 et R5 forment, ensemble avec l'atome de carbone auquel ils sont liés, un cycle carbocyclique saturé de 3-8 chaînons ;
ainsi que leurs sels pharmaceutiquement acceptables.

5. Composés de formule I selon l'une ou plusieurs des revendications 1 à 4 pour une utilisation comme médicament.

6. Médicament contenant des composés de formule I selon l'une ou plusieurs des revendications 1 à 4.

7. Médicament contenant des composés de formule I selon l'une ou plusieurs des revendications 1 à 4 et au moins une autre substance active.

8. Médicament selon la revendication 7, **caractérisé en ce qu'**il contient comme autre substance active un(e) ou plusieurs antidiabétiques, substances actives hypoglycémiantes, inhibiteurs de la HMGCoA, réductase, inhibiteurs de la résorption du cholestérol, agonistes de PPAR gamma, agonistes de PPAR alpha, agonistes de PPAR alpha/gamma, agonistes de PPAR delta, fibrates, inhibiteurs de MTP, inhibiteurs de la résorption de l'acide biliaire, inhibiteurs de CETP, adsorbants polymères de l'acide biliaire, inducteurs des récepteurs de LDL, inhibiteurs d'ACAT, antioxydants, inhibiteurs de la lipoprotéine-lipase, inhibiteurs de l'ATP-citrate-lyase, inhibiteurs de la squalène synthétase, antagonistes de la lipoprotéine (a), agonistes du récepteur de HM74A, inhibiteurs de lipase, insulines, sulfonylurées, biguanides, méglitinides, thiazolidinediones, inhibiteurs de l'α-glucosidase, substances actives agissant sur le canal calcique dépendant de l'ATP des cellules bêta, inhibiteurs de la glycogène phosphorylase, antagonistes du récepteur du glucagon, activateurs de la glucokinase, inhibiteurs de la gluconéogenèse, inhibiteurs de la fructose-1,6-biphosphatase, modulateurs du transporteur 4 du glucose, inhibiteurs de la glutamine-fructose-6-phosphate-amidotransférase, inhibiteurs de la dipeptidylpeptidase-IV, inhibiteurs de la 11-bêta-hydroxystéroïde-déshydrogénase-1, inhibiteurs de la protéine-tyrosine-phosphatase-1B, modulateurs du transporteur 1 ou 2 du glucose dépendant du sodium, modulateurs du GPR40, inhibiteurs de la lipase sensible aux hormones, inhibiteurs de l'acétyl-CoA carboxylase, inhibiteurs de la phosphoénolpyruvate-carboxykinase, inhibiteurs de la glycogène synthase kinase-3 bêta, inhibiteurs de la protéine kinase C bêta, antagonistes du récepteur de l'endothéline-A, inhibiteurs de la I kappaB kinase, modulateurs du récepteur des glucocorticoïdes, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes ß3, antagonistes du récepteur de CB1, agonistes de MSH (hormone stimulant les mélanocytes), agonistes de CCK, inhibiteurs de la réabsorption de la sérotonine, composés sérotoninergiques et noradrénergiques mixtes, agonistes de 5HT, agonistes de bombésine, agonistes de galanine, hormones de croissance, composés libérant l'hormone de croissance, agonistes de TRH, modulateurs de la protéine découplante 2 ou 3, agonistes de leptine, agonistes de DA, inhibiteurs de lipase/amylase, modulateurs de PPAR, modulateurs de RXR ou agonistes de TR-ß ou amphétamines.

9. Procédé pour la préparation d'un médicament contenant les composés de formule I selon l'une ou plusieurs des revendications 1 à 4, **caractérisé en ce que** la substance active est mélangée avec un support pharmaceutiquement approprié et ce mélange est amené dans une forme appropriée pour l'administration.

10. Utilisation des composés de formule I selon l'une ou plusieurs des revendications 1 à 4 pour la préparation d'un médicament destiné au traitement de l'hyperglycémie.

11. Utilisation des composés de formule I, selon l'une ou plusieurs des revendications 1 à 4 pour la préparation d'un médicament destiné au traitement du diabète.

12. Utilisation des composés de formule I selon l'une ou plusieurs des revendications 1 à 4 pour la préparation d'un médicament destiné au traitement de la résistance à l'insuline.

13. Kit constitué par des emballages séparés de
a) une quantité efficace d'un composé de formule I selon l'une ou plusieurs des revendications 1 à 4 et
b) une quantité efficace d'une autre substance médicamenteuse.
